# EUROPEAN PATENT APPLICATION

(11) **EP 4 317 186 A1**
(43) Date of publication of application: **07.02.2024**
(21) Application number: 22774246.7
(22) Date of filing: 22.03.2022
(51) Int. Cl.: C07K 16/28, C12N 15/13, C12N 5/10, A61P 35/00, A61K 39/395

(54) **NANOBODY TARGETING BCMA AND APPLICATION THEREOF**

(30) Priority: 22.03.2021 CN 202110301079
(71) Applicant: Zhejiang Nanomab Technology Center Co. Ltd., Changle Township Shengzhou Shaoxing City Zhejiang Province, 312467 (CN)
(72) Inventor: PENG, Bo, Shanghai 201805 (CN); LI, Jiaguo, Shanghai 201805 (CN); YU, Haixiang, Shanghai 201805 (CN); LIU, Xiangzhen, Shanghai 201805 (CN); SUN, Yan, Shanghai 201805 (CN); ZHU, Weimin, Shanghai 201805 (CN); QIAN, Qijun, Shanghai 201805 (CN)
(74) Representative: Cabinet Beau de Loménie
(86) International application number: PCT/CN2022/082351
(87) International publication number: WO 2022/199590

(57) **Abstract**

Disclosed in the present invention is a nanobody, comprising a heavy chain variable region containing CDR1, CDR2 and/or CDR3, and having the function of recognizing and binding to BCMA. Reference can be made to the text for specific CDR sequences. Also disclosed in the present invention are a BCMA binding molecule, a composition comprising the nanobody or BCMA binding molecule, a BCMA detection agent, and an application of the nanobody, the BCMA binding molecule, or the composition in preparation of a drug for treating a BCMA related disease. The nanobody, BCMA binding molecule, and composition in the present invention can recognize and bind to BCMA, and have potential utility for treatment of BCMA related diseases.

## Description

### Technical Field

The invention belongs to the field of biopharmacy, and relates to a nanobody targeting BCMA and use thereof.

### Technical Background

Single-domain antibodies (sdAbs) differ from traditional 4-chain antibodies by having a single monomeric antibody variable domain. For example, camelids and sharks produce antibodies that naturally lack of light chains, which are called heavy chain-only antibodies (HcAbs, or heavy chain antibodies). Antigen-binding fragments in each arm of camelid heavy chain-only antibodies have a single heavy chain variable domain (VHH) that can have high affinity for antigen without the help of a light chain. Camelid VHHs or nanobodies are known as the smallest functional antigen-binding fragments, with a molecular weight of about 15kD.

Nanobodies have the natural advantages of high stability, strong penetration, and being able to bind to a wide range of epitopes (Muyldermans S. AnnuRev Biochem. 2013; 82:775-97). Since being discovered, nanobodies have gradually attracted attention, and the basic research thereon has been relatively mature. Applications regarding to autoimmune diseases, blood diseases, viral infections and orthopedic diseases are under clinic trials. And nanobodies have also shown great advantages in anti-infection, anti-inflammatory diseases and treating neurodegenerative diseases (Morrison C. Nat Rev Drug Discov. 2019 Jul; 18(7): 485-487).

Multiple myeloma (MM) is the second most common hematopoietic malignancy, accounting for 10% of hematological malignancies. The main pathology of MM is the indefinite expansion and enrichment of plasma cells in the bone marrow (BM), leading to osteonecrosis. Patients affected by MM may experience a variety of disease-related symptoms due to bone marrow infiltration, bone destruction, renal failure, immunodeficiency, and the psychological burden of the cancer. At present, the main treatment options are chemotherapy and stem cell transplantation. Medicaments for chemotherapy are mainly steroids, thalidomide, lenalidomide, bortezomib or a combination of various cytotoxic agents. For younger patients, high-dose chemotherapy combined with autologous stem cell transplantation can be used. However, a major problem is MM disease relapse due to the continuous emergence and evolution of drug-resistant clones, and significant adverse effects are unavoidable. Therefore, more effective and less toxic treatments are urgently needed to overcome drug resistance, improve quality of life, and prolong survival in patients with relapsed and refractory MM (Cho, S.-F., Frontiers in Immunology, 9).

BCMA (B-cell maturation antigen) is a B cell maturation antigen, a type III transmembrane protein composed of 184 amino acid residues, belonging to the TNF receptor superfamily. BCMA's ligands belong to the TNF superfamily, such as proliferation-inducing ligand (APRIL), B lymphocyte stimulating factor (BAFF). When binding to its ligand, BCMA can activate NF-κB and MAPK8/JNK signaling to mediate the proliferation and survival of B cells, which plays an important role in the survival of long-lived plasma cells. BCMA is specifically highly expressed on the surface of advanced B cells, short-lived proliferating plasmablasts and long-lived plasma cells, and exists on memory B cells to a certain extent, but has not been reported to be found in naive B cells, CD34-positive hematopoietic stem cells and other normal tissue cells. Studies have shown that the expression of BCMA is associated with hematological malignancies, most notably multiple myeloma (MM); The overexpression of BCMA is related to the occurrence, development and poor prognosis of MM. Therefore, BCMA is an ideal target for MM targeted therapy (Tai, Immunotherapy, 7(11), 1187-1199). High affinity antibodies can block the binding between BCMA and its natural ligands BAFF and APRIL. Anti-BCMA sdAbs can be used in combination with cellular immunotherapy of CAR-T cells, for example, to enhance cytotoxicity against tumor cells. There is currently a lack of anti-BCMA nanobodies with good efficacy and low toxicity.

### Summary of the Disclosure

In order to overcome the deficiencies of lacking BCMA targeting nanobodies with good effect and low toxicity in the prior art, the present invention provides a BCMA targeting nanobody and use thereof.

In order to solve the above-mentioned technical problems, the first aspect of the technical solution of the present invention is: a nanobody, which comprises a heavy chain variable region containing CDR1, CDR2 and CDR3, and has the function of recognizing and binding BCMA, and:
Wherein, the CDR1 is selected from the following group:
(1) as shown by the amino acid sequence of SEQ ID NO: 4, or having substitution, deletion or addition of 1 to 3 amino acid residues in the amino acid sequence shown by SEQ ID NO: 4;
(2) as shown by the amino acid sequence of SEQ ID NO: 6, or having substitution, deletion or addition of 1 to 3 amino acid residues in the amino acid sequence shown by SEQ ID NO: 6;

the CDR2 is shown by the amino acid sequence of SEQ ID NO: 23, or has substitution, deletion or addition of 1 to 3 amino acid residues in the amino acid sequence shown by SEQ ID NO: 23;
the CDR3 is selected from the amino acid sequences shown by SEQ ID NOs: 27-52.

Preferably, when the CDR1 has a substitution of 1 to 3 amino acid residues in the amino acid sequence shown by SEQ ID NO: 4, the substitution is selected from G1E , T3I, S5R and S6P/I; when the CDR1 has a substitution of 1 to 3 amino acid residues in the amino acid sequence shown by SEQ ID NO: 6, the substitution is selected from F4S/D, S5R and I6F; and/or, when the CDR2 is a substitution of 1 to 3 amino acid residues in the amino acid sequence shown by SEQ ID NO: 23, the substitution is selected from the group consisting of I1V, Y2T, S3P/G/T, D4G/E/A, G5S/N, S6R/N/G/T and T7A/P/S.

In a preferred specific embodiment, the CDR1 is selected from the amino acid sequences shown by SEQ ID NOs: 2-7; the CDR2 is selected from the amino acid sequences shown by SEQ ID NOs: 8-26; and/or, the CDR3 is selected from the amino acid sequences shown by SEQ ID NOs: 27-52.

In a more preferred embodiment, the heavy chain variable region comprises CDR1, CDR2 and CDR3 shown by any one group of the following groups 1 to 26:

| Gro up | Antibody No. | CDR1 (SEQ ID NO) | CDR2 (SEQ ID NO) | CDR3 (SEQ ID NO) |
|---|---|---|---|---|
| 1 | NB-27 | 7 | 9 | 46 |
| 2 | NB-29 | 4 | 23 | 33 |
| 3 | NB-34 | 6 | 25 | 44 |
| 4 | NB-36 | 4 | 19 | 51 |
| 5 | NB-51 | 4 | 18 | 50 |
| 6 | NB-53 | 3 | 19 | 30 |
| 7 | NB-65 | 4 | 23 | 29 |
| 8 | NB-67 | 6 | 11 | 47 |
| 9 | NB-71 | 4 | 21 | 52 |
| 10 | NB-79 | 3 | 14 | 39 |
| 11 | NB-82 | 6 | 26 | 45 |
| 12 | NB-83 | 7 | 8 | 49 |
| 13 | NB-84 | 4 | 20 | 31 |
| 14 | NB-90 | 3 | 13 | 32 |
| 15 | NB-100 | 4 | 20 | 37 |
| 16 | NB-102 | 3 | 17 | 41 |
| 17 | NB-122 | 4 | 23 | 28 |
| 18 | NB-170 | 2 | 12 | 38 |
| 19 | NB-192 | 4 | 23 | 27 |
| 20 | NB-216 | 6 | 24 | 48 |
| 21 | NB-217 | 3 | 23 | 35 |
| 22 | NB-222 | 5 | 10 | 43 |
| 23 | NB-257 | 3 | 22 | 40 |
| 24 | NB-265 | 4 | 23 | 34 |
| 25 | NB-352 | 3 | 15 | 36 |
| 26 | NB-367 | 3 | 16 | 42 |

In a more preferred embodiment, the amino acid sequence of the heavy chain variable region is shown by SEQ ID NOs: 53-78, or have at least 80%, 90%, 95%, 96%, 97%, 98% or 99% identity to the amino acid sequences shown by SEQ ID NOs: 53-78.

In some embodiments, the description also provides a nanobodies, heavy chain antibodies, antibodies or antigen binding fragment thereof that bind to the same epitope of BCMA as any nanobody of the description, that is, nanobodies, heavy chain antibodies, antibodies or antigen binding fragment thereof that can compete with any nanobody of the description for binding to BCMA.

In order to solve the above-mentioned technical problems, the second aspect of the technical solution of the present invention is: a BCMA binding molecule, wherein the BCMA binding molecule comprises the nanobody as described in the first aspect, for example, the BCMA binding molecule is a monovalent or multivalent nanobody, a multispecific nanobody, a heavy chain antibody or an antigen-binding fragments thereof comprising one, two or more the nanobodies according to the first aspect.

In a preferred embodiment, the BCMA binding molecule is a heavy chain antibody.

In a more preferred embodiment, the amino acid sequence of the CH2 and CH3 of the heavy chain is shown by SEQ ID NO: 1, or have at least 80%, 90%, 95%, 96%, 97%, 98% or 99% identity to the amino acid sequences shown by SEQ ID NO: 1.

In the present invention, the term "nanobody" represents the variable region of a heavy chain antibody (heavy chain variable region). Nanobodies, VHHs, single domain antibodies (sdAbs) represent the same molecule in the present invention. The term "heavy chain antibody" includes nanobodies together with the CH2 and CH3 regions of conventional heavy chains. The term "BCMA binding molecule" can be a nanobody, a heavy chain antibody, or a multivalent nanobody/heavy chain antibody comprising one, two or more nanobodies of the same specificity, multispecific antibodies comprising two or more nanobodies of the different specificities, conventional antibodies or antigen-binding fragments thereof, etc.

In order to solve the above technical problems, the third aspect of the technical solution of the present invention is: an isolated nucleic acid, wherein the isolated nucleic acid encodes the nanobody as described in the first aspect of the present invention, or the BCMA binding molecules as described in the second aspect of the present invention.

In order to solve the above technical problems, the fourth aspect of the technical solution of the present invention is: a recombinant expression vector, wherein the recombinant expression vector comprises the isolated nucleic acid according to the third aspect of the present invention. Preferably, the backbone of the recombinant expression vector is pCDNA3.4.

In order to solve the above-mentioned technical problems, the fifth aspect of the technical solution of the present invention is: a transformant, wherein the transformant comprises the isolated nucleic acid according to any one of the third aspects of the present invention, or the recombinant expression vector according to any one of the four aspects of the present invention. Preferably, the host of the transformant is a prokaryotic cell or a eukaryotic cell. More preferably, the eukaryotic cell is a CHO cell.

In order to solve the above-mentioned technical problems, the sixth aspect of the technical solution of the present invention is: a composition, comprising one, two or more nanobodies according to the first aspect of the present invention, the BCMA binding molecule according to the second aspect of the present invention, the isolated nucleic acid according to the third aspect of the present invention, the recombinant expression vector according to the fourth aspect of the present invention, or the transformant according to the fifth aspect of the present invention. Preferably, the composition further includes pharmaceutically acceptable excipients, so that the composition is a pharmaceutical composition.

In order to solve the above technical problems, the seventh aspect of the technical solution of the present invention is: a BCMA detection agent, wherein the BCMA detection agent comprises the nanobody according to any one of the first aspect of the present invention and/or the BCMA binding molecule according to any one of the second aspect of the present invention; preferably, the BCMA detection agent is used for flow cytometry.

In order to solve the above-mentioned technical problems, the eighth aspect of the technical solution of the present invention is: the use of the nanobody according to the first aspect of the present invention, the BCMA binding molecule according to the second aspect of the present invention, the isolated nucleic acid according to the third aspect of the present invention, the recombinant expression vector according to the fourth aspect of the present invention or the transformant according to the fifth aspect of the present invention in the preparation of medicaments for the treatment of a BCMA related disease. The disease is preferably a hematological malignancy, more preferably multiple myeloma.

In order to solve the above-mentioned technical problems, the ninth aspect of the technical solution of the present invention is: a method for treating a subject in need, comprising administered the nanobody according to the first aspect of the present invention, the BCMA binding molecule according to the second aspect of the present invention, the isolated nucleic acid according to the third aspect of the present invention, the recombinant expression vector according to the fourth aspect of the present invention, the transformant according to the fifth aspect of the present invention, or the composition according to the sixth aspect of the present invention to the subject in need thereof; wherein the subject in need thereof has a BCMA related disease. The disease is preferably a hematological malignancy, more preferably multiple myeloma.

The above preferred conditions can be combined arbitrarily to obtain preferred examples of the present invention, as long as the combination comply with common knowledge in the art.

The reagents and raw materials used in the present invention are all commercially available.

The positive progressive effect of the present invention is:
1. The nanobody, the BCMA binding molecule and the composition provided by the invention can recognize and bind to BCMA, and have potential effects of treating diseases related to BCMA.
2. The BCMA detection agent of the present invention can detect BCMA rapidly and efficiently.

### Description of Figures

Figure 1 shows the detection of titer of alpaca serum; A and B in Fig. 1 are the serum titers of A1 and A2 alpaca after different immunizations, respectively;
Figure 2 shows the binding of antigens and IgG subtypes purified by serum before and after immunization of alpaca; A and B of Figure 2 show the changes of IgG subtypes in serum before and after immunization of A1 alpaca and A2 alpaca respectively;
Figure 3 is the binding of clone supernatant and BCMA protein detected by ELISA;
Figure 4 is the reconstruction structure of nanobody (VHH);
Figure 5 shows a ligand competition binding experiment at the CHO-K1/BCMA cell level; A in Figure 5 is MFI and IC50 of NB-67, NB-84, NB-90, NB-100, NB-122, NB-192, NB-257, NB-265 and NB-367, B in Figure 5 is MFI and IC50 of NB-27, NB-29, NB-34, NB-36, NB-51, NB-53, NB-65, NB-71, NB -83, NB-102, NB-216, NB-217, NB-170, NB-222 andNB-352;
Figure 6 shows the affinity detection at the CHO-K1/BCMA cell level; wherein the BMK2-H4 is the positive control antibody; A in Figure 6 is MFI and EC50 of NB-1, NB-27, NB-29, NB-34, NB-36, NB- 51, NB-53, NB-65, NB-67, NB-71, NB-82, NB-83 and NB-84, B in Figure 6 is MFI and EC50 of NB-90, NB-100, NB-102, NB-122, NB-192, NB-216, NB-217, NB-257, NB-265 and NB-367;
Figure 7 shows the detection of the affinity level of the antibody of the present invention binding to target cells; wherein A, B and C in Figure 7 are the binding affinity levels of each antibody to RPMI8226 cells, Jeko-1 cells and Daudi cells respectively; BMK1 and BMK2-H4 are positive control antibody, Isotype1 and Isotype2 are isotype control antibodies (i.e., isotype antibodies), the source and sequence of the control antibody are shown in Table 6, and EC50 is shown in nM;
Figure 8 shows a flow chart of MPA screening, and the detection results of 4 antibodies; wherein A in Figure 8 is the MPA screening process, and B, C, D, and E in Figure 8 are MPA assay results of NB-1, NB-29, NB-257, and NB-367 antibody.

### Detailed Description

### Antibody

"BCMA binding molecule" as used herein is a protein that has the function of recognizing and binding BCMA, including but not limited to antibodies, antigen binding fragments of antibodies, heavy chain antibodies, nanobodies, micro antibodies, affibodies, target binding regions of receptors, cell adhesion molecules, ligands, enzymes, cytokines, and chemokines.

Term "antibody" as used herein includes monoclonal antibodies (including full-length antibodies with immunoglobulin Fc region), antibody compositions with polyepitopic specificity, multi-specific antibodies (e.g., bispecific antibodies), diabodies and single chain molecules, and antibody fragments, especially antigen binding fragments, (e.g., Fab, F(ab')2, and F_{V}). In some embodiments herein, the term "immunoglobulin" (Ig) is used interchangeably with "antibody" herein.

The basic 4-chain antibody unit is a heterotetrameric glycoprotein composed of two identical light chains (L) and two identical heavy chains (H). IgM antibody consists of 5 basic heterotetramer units and another polypeptide called J chain, which contains 10 antigen binding sites; IgA antibody contains 2-5 basic 4 chain units, which can polymerize with J chain to form a multivalent assemblages. In the case of IgGs, the 4-chain unit is typically about 150,000 daltons. Each light chain is connected to a heavy chain through a covalent disulfide bond, while the two heavy chains are connected to each other through one or more disulfide bonds, and the number of disulfide bonds depends on the isotype of the heavy chain. Each heavy and light chain also has an interchain disulfide bridge with regular spacing. Each heavy chain has a variable domain (VH) at the N-terminus, followed by three constant domains (for each α and γ chain, CH1, CH2, and CH3) and four constant domains (for µ and ε isoforms, CH1, CH2, CH3, and CH4) and the hinge region (Hinge) between the CH1 domain and the CH2 domain. Each light chain has a variable domain (VL) at the N-terminus, followed by a constant domain (CL) at the other end. VL is aligned with VH, while CL is aligned with the first constant domain (CH1) of the heavy chain. Specific amino acid residues are thought to form an interface between light and heavy chain variable domains. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called kappa and lambda, based on the amino acid sequences of their constant domains. Depending in the amino acid sequence of the constant domain of their heavy chains (CH), immunoglobulins can be assigned to different classes or isotypes. There are five classes of immunoglobulins: IgA, IgD, IgE, IgG and IgM, having heavy chains designated α, δ, ε, γ and µ, respectively. The γ and α classes are further divided into subclasses on the basis of relatively minor differences in the CH sequence and function, e.g., humans express the following subclasses: IgGl, IgG2A, IgG2B, IgG3, IgG4, IgA1 and IgA2.

"Heavy chain antibody" described herein is an antibody derived from camelidae or sharks. Compared with the above 4-chain antibody, the heavy chain antibody lacks light chain and heavy chain constant region 1 (CH1), and only contains two heavy chains composed of variable region (VHH) and other constant regions, wherein the variable region is connected to the constant region through a hinge region like structure. Each heavy chain of camelid heavy chain antibody contains one variable region (VHH) and two constant regions (CH2 and CH3), and each heavy chain of shark heavy chain antibody contains one variable region and five constant regions (CH1-CH5). Antigen binding fragments of heavy chain antibodies include VHH and single chain heavy chain antibodies. Heavy chain antibodies may have CH2 and CH3 of human IgG Fc by fusing with the constant region of human IgG Fc.

As used herein, the terms "single domain antibody", "anti-BCMA single domain antibody", "heavy chain variable region domain of heavy chain antibody", "VHH" and "nanobody" can be used interchangeably, and all refer to nanobodies that specifically recognize and bind to BCMA. Nanobodies are variable regions of heavy chain antibodies. Typically, nanobodies contain three CDRs and four FRs. Nanobodies are the smallest functional antigen binding fragments. Generally, after obtaining an antibody which naturally lacks light chain and heavy chain constant region 1 (CH1), the heavy chain variable region of the antibody is cloned to construct a nanobody consisting of only one heavy chain variable region.

Binding molecules comprising two or more nanobodies are multivalent nanobodies; and binding molecules comprising two or more nanobodies with different specificities are multispecific nanobodies. Multivalent nanobodies or multispecific nanobodies are connected to multiple nanobodies through linkers. The linker usually consists of 1-15 amino acids selected from G and S, for example (G₄S)₃.

The terms "heavy chain antibody" and "antibody" herein are intended to distinguish different composition forms of antibodies. Due to the similarity of their structures, the following descriptions on structures of antibodies except for light chains also apply to heavy chain antibodies.

The "variable region" or "variable domain" of an antibody refers to the amino-terminal domains of the heavy or light chain of the antibody. The variable domains of the heavy chain and light chain may be referred to as "VH" and "VL", respectively. These domains are generally the most variable parts of the antibody (relative to other antibodies of the same class) and contain the antigen binding sites.

The term "variable" refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains, namely HCDR1, HCDR2 and HCDR3 of heavy chain variable region (CDR1, CDR2 and CDR3 in heavy chain antibodies for short) and LCDR1, LCDR2 and LCDR3 of light chain variable region. The more highly conserved portions of variable domains are called the framework regions (FR). The variable domains of native heavy and light chains each comprise four FR regions (FR1, FR2, FR3, and FR4), largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domains are not involved directly in the binding of antibody to an antigen, but exhibit various effector functions, such as participation of the antibody in antibody-dependent cellular toxicity.

An "Fc region" (fragment crystallizable region) or "Fc domain" or "Fc" refers to the C-terminal region of the heavy chain of an antibody that mediates the binding of the immunoglobulin to host tissues or factors, including binding to Fc receptors located on various cells of the immune system (e.g., effector cells) or to the first component (Clq) of the classical complement system. In the IgG antibody isotype, the Fc region consists of two identical protein fragments derived from the CH2 and CH3 domains of the two heavy chains of the antibody. Although the boundaries of the Fc region of an immunoglobulin heavy chain might vary, the human IgG heavy chain Fc region is usually defined to stretch from an amino acid residue at position C226 or P230 to the carboxy-terminus of the heavy chain, wherein the numbering is according to the EU index as in Kabat. As used herein, the Fc region may be a native sequence Fc or a variant Fc.

An "antibody fragment" comprises a portion of an intact antibody, preferably the antigen binding and/or the variable region of the intact antibody. The antibody fragment is preferably an antigen binding fragment of the antibody. Examples of antibody fragments include Fab, Fab', F(ab')2 and Fv fragments; diabodies; linear antibodies; single-chain antibody molecules, scFv-Fc fragment; multispecific antibodies formed from antibody fragments; and any fragment that should be able to increase the half-life by chemical modification or by incorporation into liposomes. Papain digestion of antibodies produced two identical antigen-binding fragments, called "Fab" fragments, and a residual "Fc" fragment, a designation reflecting the ability to crystallize readily. The Fab fragment consists of an entire L chain along with the variable region domain of the H chain (VH), and the first constant domain of one heavy chain (CH1). Each Fab fragment is monovalent with respect to antigen binding, i.e., it has a single antigen-binding site. Pepsin treatment of an antibody yields a single large F(ab')2 fragment which roughly corresponds to two disulfide linked Fab fragments having different antigen-binding activity and is still capable of cross-linking antigen. Fab' fragments differ from Fab fragments by having a few additional residues at the carboxy terminus of the CH1 domain including one or more cysteines from the antibody hinge region. F(ab')2 antibody fragments originally were produced as pairs of Fab' fragments which have hinge cysteines between them. Other chemical couplings of antibody fragments are also known. The Fc fragment comprises the carboxy-terminal portions of both H chains held together by disulfides. The effector functionfunctions of an antibody isantibodies are determined by the sequencesequences in the Fc region, the region which is also recognized by Fc receptors (FcRsFcR) found on somecertain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site. "Single-chain Fv " also abbreviated as "sFv " or "scFv " are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method, phage-display technologies, recombinant DNA methods, and technologies for producing human or humanlike antibodies in animals that have parts or all of the human immunoglobulin loci or genes encoding human immunoglobulin sequences, single-cell sequencing methods.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity.

"Humanized" forms of non-human (e.g., murine) antibodies are chimeric antibodies that contain minimal sequence derived from non-human immunoglobulin. Therefore, "humanized antibodies" generally refer to non-human antibodies that have had the variable- domain framework regions swapped for sequences found in human antibodies. Generally, in a humanized antibody, the entire antibody, except the CDRs, is encoded by a polynucleotide of human origin or is identical to such an antibody except within its CDRs. The CDRs, some or all of which are encoded by nucleic acids originating in a non-human organism, are grafted into the beta-sheet framework of a human antibody variable region to create an antibody, the specificity of which is determined by the engrafted CDRs. The creation of such antibodies are well known in the art, such as using mice with genetically engineered immune systems. In the description, antibodies, nanobodies, heavy chain antibodies, etc. all include humanized variants of the antibodies.

A "human antibody" is an antibody that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries.

The invention also includes derivatives and analogs of the various antibodies (e.g., nanobodies, heavy chain antibodies or antigen-binding fragments thereof, multivalent nanobodies, multispecific nanobodies, antibodies or antigen-binding fragments thereof). "Derivatives" and "analogues" refer to polypeptides that basically maintain the same biological function or activity of the various antibodies of the present description. The derivatives or analogues of the present description may be polypeptides formed from (i) a polypeptide with a substituent group in one or more amino acid residues, or (ii) a polypeptide formed from fusion of a mature polypeptide with another compound, such as a compound that extends the half-life of the polypeptide, such as polyethylene glycol, or (iii) a polypeptide formed by fusing an additional amino acid sequence to this polypeptide sequence (such as a leader sequence or a secretory sequence, or a sequence or prokaryotic sequence used for purifying this polypeptide, or a fusion protein formed with a 6His tag). According to the teaching herein, these derivatives and analogues belong to common sense known to those skilled in the art.

Without substantially affecting the activity of the antibody, those skilled in the art may change one or more (for example, 1, 2, 3, 4, 5, 6, 7, 8, 9 or 10 or more) amino acids to the sequence of the description to obtain the variant of the antibody or the functional fragment sequence thereof. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In this field, conservative substitution with amino acids with similar or similar properties usually does not change the function of the protein. For example, substituting with amino acids having similar properties may be performed in the FR and/or CDR regions of the variable region. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present description.

The variant forms of the various antibodies described herein include: homologous sequence, conservative variant, allelic variant, natural mutant, induced mutant, protein encoded by DNA that can hybridize with the coding DNA of the various antibody of the description under high or low strictness conditions, and polypeptide or protein obtained by using the antiserum of the various antibody of the description.

In some embodiments, the sequence of the variant of the present description may have at least 95%, 96%, 97%, 98% or 99% identity to its source sequence. The sequence identity described in the description can be measured using sequence analysis software. For example, the computer program BLAST with default parameters, especially BLASTP or TBLASTN. The description also comprises those molecules with variable regions of antibody heavy chain with CDRs, if their CDRs have more than 90% homology (preferably more than 95%, more preferably more than 98%) with the CDRs identified here.

The antibody of the description can be prepared by conventional methods in the art, such as hybridoma technology well known in the art. The nanobody and the heavy chain antibody of the description can be prepared by conventional methods in the art, such as phage display technology well known in the art. Alternatively, the various antibodies of the present description may be expressed in other cell lines. Suitable mammalian host cells can be transformed with sequences encoding the various antibodies of the present description. Transformation can be carried out using any known method, including, for example, packaging polynucleotides in viruses (or viral vectors) and transducing host cells with the viruses (or vectors). The transformation procedure used depends on the host to be transformed. Methods for introducing heterologous polynucleotides into mammalian cells are well known in the art, including dextran mediated transfection, calcium phosphate precipitation, Polybrene mediated transfection, protoplast fusion, electroporation, encapsulation of polynucleotides in liposomes and direct microinjection of DNA into the nucleus. Mammalian cell lines that can be used as hosts for expression are well known in the art, including but not limited to a variety of immortalized cell lines available from the American Typical Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., HepG2), etc. Particularly preferred cell lines are selected by determining which cell lines have high expression levels and produce antibodies with substantial BCMA binding properties.

### Nucleic acid

The description also provides polynucleotides encoding the above various antibody or fragments thereof. Polynucleotides encoding heavy chain variable region, light chain variable region, heavy chain, light chain and CDRs are provided. The polynucleotide of the description can be in the form of DNAs or RNAs. DNAs include cDNAs, genomic DNAs, or synthetic DNAs. DNAs can be single stranded DNAs or double stranded DNAs. DNAs can be coding or noncoding strand DNAs.

As those skilled in the art will understand, due to the degeneracy of the genetic code, an extremely large number of nucleic acids can be prepared, all of which encode the antibody of the description or antigen binding fragment thereof. Therefore, when a specific amino acid sequence has been identified, those skilled in the art can simply modify the sequence of one or more codons without changing the amino acid sequence of the encoded protein to produce any number of different nucleic acids. Therefore, the present description also relates to polynucleotides that hybridize with the above polynucleotide sequences and have at least 50%, preferably at least 70%, more preferably at least 80% identity between the two sequences. The description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. Moreover, the polypeptides encoded by hybridizable polynucleotides have the same biological functions and activities as mature polypeptides.

The nucleotide full-length sequence of the various antibodies of the description or fragment thereof can usually be obtained by PCR amplification method, recombination method or artificial synthesis method. A feasible method is to synthesize relevant sequences by artificial synthesis, especially with short fragment length. Usually, fragments with very long sequence can be obtained by synthesizing several small fragments first and then connecting them. In addition, the coding sequence of the heavy chain and the expression tag (such as 6His) can also be fused together to form a fusion protein.

Once the relevant sequences are obtained, they can be obtained in large quantities by recombination. They are related sequences usually cloned into vectors, transferred into cells, and then isolated from the proliferated host cells by conventional methods. The biomolecules (nucleic acids, proteins, etc.) according to the present description include biomolecules in isolated form. At present, the DNA sequence encoding the protein (or fragment thereof, or derivative thereof) of the present disclosure can be obtained completely through chemical synthesis. This DNA sequence can then be introduced into various existing DNA molecules (or such as vectors) and cells known in the art. In addition, mutations can also be introduced into the protein sequence of the present disclosure through chemical synthesis.

Therefore, the present description also relates to nucleic acid constructs, such as expression vectors and recombinant vectors, comprising the above appropriate DNA sequence and the appropriate promoter or control sequence. These vectors can be used to transform appropriate host cells to enable them to express proteins. Vectors usually contain sequences for plasmid maintenance and for cloning and expressing exogenous nucleotide sequences. The sequences (collectively referred to as "flanking sequence" in some embodiments) generally comprises one or more of the following nucleotide sequences: promoter, one or more enhancer sequences, replication origin, transcription termination sequence, complete intronic sequence comprising donor and receptor splice sites, sequence encoding leader sequence for polypeptide secretion, ribosome binding site, polyadenylation sequence, a multi-linker region for inserting nucleic acids encoding antibodies to be expressed and an optional marker element.

Host cells can be prokaryotic cells, such as bacterial cells; or lower eukaryotic cells, such as yeast cells; or higher eukaryotic cells, such as mammalian cells. Representative examples are: Escherichia coli, Streptomyces; bacterial cells of Salmonella typhimurium; fungal cells such as yeast; insect cells of Drosophila S2 or Sf9; animal cells of CHO, COS7, 293 cells, etc.

In some embodiments, host cells may be various functional cells well known in the art, such as various killer cells, including but not limited to cytokine induced killer cells (CIK), dendritic cell stimulated cytokine induced killer cells (DC-CIK), cytotoxic T lymphocytes (CTL), γδ T cells, natural killer cells (NK), tumor infiltrating lymphocytes (TIL), lymphokine activated killer cells (LAK), CD3AK cells (killer cells of anti-CD3 mAb), and CAR-T/TCR-T cells. In some embodiments, the killer cells are T cells or NK cells. Exemplary NK cells include, but are not limited to, primary NK cells, NK cell strains (such as NK92), and NKT cells. In some embodiments, the NK cells are primary NK cells. Exemplary T cells include, but are not limited to, peripheral blood T lymphocytes, cytotoxic T cells (CTLs), helper T cells, inhibitory/regulatory T cells, yδ T cells and mixed T cell populations of cytokine induced killer cells (CIK) and tumor infiltrating lymphocytes (TIL). In some embodiments, the T cells are peripheral blood T lymphocytes and TIL derived T cells.

Transformation of host cells with recombinant DNA can be performed using conventional techniques known to those skilled in the art. When the host is a prokaryote such as Escherichia coli, competent cells that can absorb DNA can be harvested after the exponential growth period and treated with CaCl₂ method, the steps of which are well known in the art. Another method involves the use of MgCl₂. If necessary, the transformation can also be performed by electroporation. When the host is eukaryote, the following DNA transfection methods can be selected: calcium phosphate coprecipitation method, conventional mechanical methods such as microinjection, electroporation, liposome packaging, etc.

The obtained transformants can be cultured by conventional methods to express the polypeptide encoded by the gene of the description. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

The polypeptide in the above method can be expressed inside the cell, on the cell membrane, or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods.

### Use of treatment and pharmaceutical composition

All aspects of the various antibodies described herein can be used to prepare medicaments to prevent or treat various conditions and diseases described herein, especially those related to BCMA expressing cells. In some embodiments, the conditions and diseases are cancers, preferably hematological malignancies, more preferably multiple myeloma.

The pharmaceutical composition herein comprises the binding molecules described herein, as well as pharmaceutically acceptable excipients, including but not limited to diluents, vehicles, solubilizers, emulsifiers, preservatives, and/or adjuvants. The excipients are preferably non-toxic to the recipient at the dose and concentration used. Such excipients include (but are not limited to): saline, buffer, glucose, water, glycerol, ethanol, and their combinations. In some embodiments, the pharmaceutical composition may contain substances for improving, maintaining, or retaining, for example, the pH, permeability, viscosity, clarity, color, isotonicity, odor, sterility, stability, dissolution or release rate, absorption, or permeation of the composition. These substances are known in the prior art. The optimal pharmaceutical composition can be determined according to the expected route of administration, mode of delivery, and required dose.

Pharmaceutical compositions for in vivo administration are usually provided in the form of sterile formulations. Sterilization is achieved by filtration through a sterile filter membrane. When the composition is lyophilized, this method can be used for sterilization before or after lyophilization and rehydration. The pharmaceutical composition of the present description may be selected for parenteral delivery. Compositions for parenteral administration may be in lyophilized form or stored in solution. For example, it is prepared by conventional methods with normal saline or aqueous solution comprising glucose and other adjuvants. Parenteral compositions are usually placed in containers with sterile access holes, such as intravenous solution strips or vials with plugs that can be pierced by subcutaneous injection needles. Alternatively, compositions may be selected for inhalation or delivery through the digestive tract, such as oral. The preparation of the pharmaceutically acceptable composition is within the art. Other pharmaceutical compositions will be apparent to those skilled in the art, including formulations comprising antibodies in sustained or controlled release delivery formulations. The techniques used to prepare a variety of other sustained or controllable delivery modes (such as liposome carriers, bioerodible particles or porous beads, and deposit injection) are also known to those skilled in the art.

Once the pharmaceutical composition is prepared, it is stored in sterile vials in the form of solution, suspension, gel, emulsion, solid, crystal or dehydrated or lyophilized powder. The formulation may be stored in ready to use form or rehydrated before administration (e.g., lyophilized). The description also provides a kit for generating a single dose administration unit. The kit of the description can each contain a first container with dried protein and a second container with aqueous formulation. In some embodiments of the present description, a kit comprising single-chamber and multi-chamber pre-filled syringes (e.g., liquid syringes and lyophilized syringes) are provided.

The present description also provides a method for treating a patient (especially a patient's BCMA related disease) by administering a binding molecule or a pharmaceutical composition thereof according to any embodiment of the present description. Terms "patient", "subject", "individual" and "object" are used interchangeably herein, including any organism, preferably animals, more preferably mammals (such as rats, mice, dogs, cats, rabbits, etc.), and most preferably humans. "Treatment" refers to the subject accepting the treatment scheme described herein to achieve at least one positive treatment effect (for example, a reduced number of cancer cells, a reduced tumor volume, a reduced rate of cancer cells infiltrating into peripheral organs, or a redeuced rate of tumor metastasis or tumor growth). The treatment scheme for effectively treating patients can vary according to many factors, such as the patient's disease status, age, weight, and the ability of the therapy to stimulate the subject's anti-cancer response.

The therapeutically effective amount of the pharmaceutical composition comprising the binding molecule of the present description to be used will depend on, for example, the degree and the target of treatment. Those skilled in the art will understand that the appropriate dose level for treatment will vary in part depending on the molecule delivered, the indication, the route of administration, and the size (body weight, body surface or organ size) and/or condition (age and general health condition) of the patient. In some embodiments, clinicians may titrate the dose and change the route of administration to obtain the optimal therapeutic effect. For example, about 10 micrograms/kg body weight per day to about 50 mg/kg body weight.

The dosing frequency will depend on the pharmacokinetic parameters of the bound molecules in the formulation used. Clinicians typically administer the compositions until a dosage that achieves the desired effect. The composition may therefore be administered as a single dose, or over time as two or more doses (which may or may not contain the same amount of the desired molecule), or as a continuous infusion through an implanted device or catheter.

The route of administration of the pharmaceutical composition is according to known methods, such as oral, intravenous, intraperitoneal, intracerebral (intraparenchymal), intraventricular, intramuscular, intraocular, intra-arterial, portal vein or intralesional injection; by continuous release system or by implantable device.

### Diagnosis, detection, and kit

The binding molecule of the present description can be used in assays due to its high avidity with BCMA, such as binding assays to detect and/or quantify BCMA expressed in tissues or cells. Binding molecules such as single domain antibodies can be used in further studies investigating the function of BCMA in disease. The methods for detecting BCMA are roughly as follows: obtaining cell and/or tissue samples; and detecting the level of BCMA in the sample.

The BCMA binding molecule of the invention can be used for diagnostic purposes to detect, diagnose or monitor diseases and/or conditions associated with BCMA. The description provides the detection of the presence of BCMA in a sample using a classical immunohistological method known to those skilled in the art. Detection of BCMA can be performed in vivo or in vitro. Examples of methods suitable for detecting the presence of BCMA include ELISA, FACS, RIA, and the like.

For diagnostic uses, binding molecules such as single domain antibodies are usually labeled with detectable labeling groups. Suitable labeling groups include (but are not limited to): radioisotopes or radionuclides (e.g., 3H, 14C, 15N, 35S, 90Y, 99Tc, 111In, 125I, 131I), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by secondary reporters (e.g., leucine zipper pair sequences, binding sites for secondary antibodies, metal binding domains, epitope tags), MRI (magnetic resonance imaging) or CT (computed tomography) contrast agents. Various methods for labeling proteins are known in the art and can be used to carry out the present description.

Another aspect of the present description provides a method for detecting the presence of a test molecule that competes with an antibody of the present description to bind BCMA. An example of one such assay would involve detecting the amount of free antibody in a solution containing an amount of BCMA in the presence or absence of the test molecule. An increase in the amount of free antibody (i.e., antibody that does not bind BCMA) will indicate that the test molecule can compete with the antibody for binding to BCMA. In one embodiment, the antibody is labeled with a labeling group. Alternatively, the test molecule is labeled, and the amount of free test molecule is monitored in the presence or absence of the antibody.

The description also provides a detection kit for detecting BCMA level. The kit comprises an antibody that recognizes BCMA protein, a lysis medium for dissolving samples, and general reagents and buffers required for detection, such as various buffers, detection markers, detection substrates, etc. The detection kit can be an in vitro diagnostic device.

The present invention is further described below by way of examples, but the present invention is not limited to the scope of the described examples. The experimental methods without specifying the specific conditions in the following examples generally used the conventional methods and conditions or followed the manufacturer's recommendation.

### Example

### Example 1. Immunization of alpaca and Immune response assays

### 1.1 Immunization of alpaca

The alpacas were immunized with recombinant human BCMA protein with a C-terminal Fc tag (Acrobiosystems, Cat. No.: BC7-H5254) purchased from Aero Company, and two alpacas (A1 and A2) were immunized. For the first immunization, 400 µg of immunogen and CFA (complete Freund's adjuvant) were mixed, and 10 points of immunization were selected subcutaneously along the scapula and back, with 200 µL per point. For the second to sixth booster immunization, IFA (Incomplete Freund's adjuvant) was used as the immune adjuvant. The second immunization was carried out at 3 weeks after the first immunization, and then one immunization every two weeks; the immunogen of the second to fifth immunization was 200 µg, and the immunogen of the sixth immunization was 100 µg. Before immunization and one week after each immunization, 10 mL of peripheral blood samples were collected, and serum samples were separated, part of which was used for immune titer detection, and the rest was frozen at -80°C.

### 1.2 IgG fractionation

IgG subclass fractionation was performed according to standard operating procedures. IgG subclasses were fractionated from alpaca serum using protein A/G resin. 5ml serum samples were loaded onto a 5ml protein G HP column and the column was washed with phosphate buffer (20mM, pH 7.4). The IgG3 (MW 90,000 Da) fraction was eluted with 0.15M NaCl, 0.58% acetic acid (pH 3.5), and the eluate was neutralized to pH 7.4 with 1M Tris-HCl (pH 8.2). Subsequently, the IgG1 (MW 150,000 Da) fraction was eluted with 0.1 M Glycine-HCl (pH 2.7), and the eluate was neutralized to pH 7.4 with 1 M Tris-HCl (pH 8.2). The effluent from the protein G HP column was then loaded onto a 5 ml protein A HP column, and the column was washed with 20 ml of phosphate buffer (20 mM, pH 7.0). The IgG2 (MW 80,000 Da) fraction was eluted with 0.15M NaCl, 0.58% acetic acid (pH 4.5), and the eluate was neutralized to pH 7.4 with 1M Tris-HCl (pH 8.2). The concentration of purified IgG1, IgG2 and IgG3 antibodies was determined by OD280 and the respective purity was assessed by reducing and non-reducing SDS-PAGE analysis.

### 1.3 Detection of immune titer:

1.3.1 The immune response of alpacas was assessed by ELISA, in which the binding of serum samples and purified IgG to the immobilized immunogen was determined. Serum collected prior to immunization and during bleeds one week after each immunization was assessed. ELISA plates were coated with 2 µg/mL human recombinant BCMA his tag diluted antigen (Acrobiosystem, BCA-H522y) overnight at 4°C; plates were then washed 3 times with wash buffer, followed by blocking for 2 hours at room temperature. The plate was then washed three times with wash buffer, and the serially diluted serum was added to the wells of the ELISA plate and incubated at 37°C for 1 hour. Plates were washed 3 times with wash buffer and incubated with anti-llama IgG HRP antibody (GenScript) for 30 minutes. After washing, TMB substrate was added to each well, and the color was developed for 3 minutes at room temperature, and then stopped with 3M HCl.

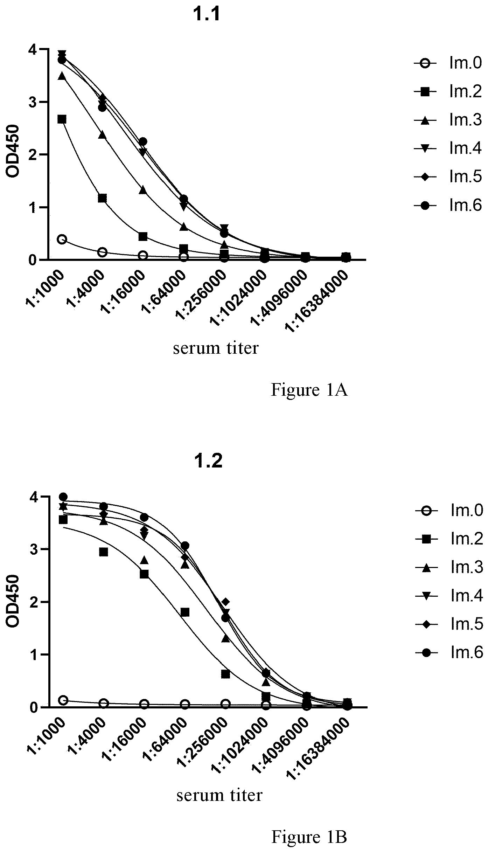

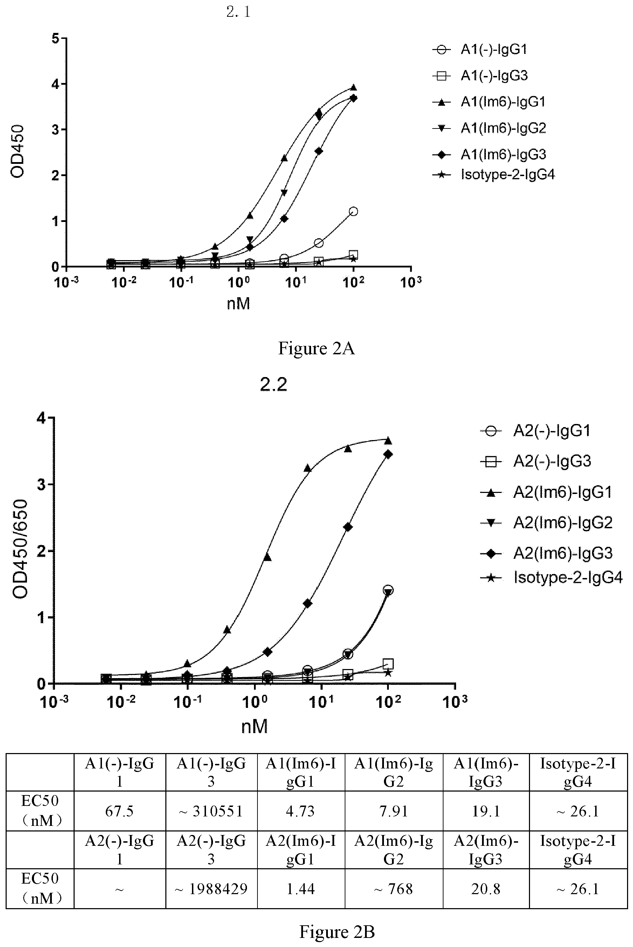

1.3.2 The experimental results showed (Figure 1-Figure 2) that after 6 immunizations, the titers of the two alpacas were maintained at a high level (titer>5 × 10⁵), which met expectations and could meet the requirement of subsequent phage library construction. A and B in Figure 1 are the serum titers of A1 and A2 alpacas after different immunizations, respectively. A and B in Figure 2 show the changes of IgG subtypes in serum before and after immunization of A1 alpaca and A2 alpaca, respectively.

### Example 2. VHH phage display library construction

### 2.1 Extraction of RNA and amplification of VHH

(1) After six immunizations, 100 mL of camelid peripheral blood lymphocytes were extracted, and total RNA was extracted. RNA was extracted according to the instructions of RNAiso reagent of Takara.
(2) The first strand of cDNA was synthesized with RNA as template and oligo dT as primer according to the instructions of reverse transcriptase of Takara.
(3) The coding genes of VHHs were obtained by nested PCR using PrimeSTAR high fidelity DNA polymerase. The variable region fragments of VHHs were amplified by nested PCR:

### First round of PCR:

Upstream primer 1: GTCCTGGCTGCTCTTCTACAAGGCC SEQ ID NO: 84)
Downstream primer 1: GGTACGTGCTGTTGAACTGTTCC (SEQ ID NO: 85)

The fragment between the heavy chain antibody guide peptide and antibody CH2 was amplified, annealed at 55°C for 30 cycles; a DNA fragment of about 600 bp was recovered as a template for the second round of PCR.

### Second round of PCR:

Upstream primer 2: GATGTGCAGCTGCAGGAGTCTGGRGGAGG (SEQ ID NO: 86)
Downstream primer 2: GGACTAGTGCGGCCGCTGGAGACGGTGACCTGG GT (SEQ ID NO: 87)

The fragment (long fragment and short fragment) between the FR1 region and the long and short hinge regions of the nanobody was amplified, annealed at 55°C for 30 cycles, and the target fragment was recovered. The result showed that the fragment was about 500 bp, that is, the nanobody gene electrophoresis band was about 500 bp.

(4) The phage pME207 and PCR amplification products were digested with Sfi I and Not I (NEB), respectively. After recovery and quantification, the two fragments were ligated with T4 DNA ligase (Takara) at a molar ratio of 1:3 and ligated overnight at 16°C.

(5) After ethanol precipitation, the ligated product was dissolved in 100 µL sterile water and electroporated into Escherichia coli TG1 in ten times. 100µL of the bacterial solution was taken after electric shock and culture, diluted by multiple ratio, coated on an ampicillin LB culture plate, the storage capacity was calculated, and the rest was coated with ampicillin 2×YT culture plate, at 37°C, invertly cultured for 13-16 h. After scraping and washing the colonies on the culture plate with 10 ml, 2×YT medium, 25% glycerol at the final concentration was added, split, and stored at -80°C for further use. The size of the library size is 4.3 × 10⁹. To detect the insertion rate of the library, 48 clones were randomly selected for colony PCR, and the results showed that the insertion rate had reached more than 90%.

(6) According to the calculated library capacity results, viable cells with 10 times the library capacity were seeded in 200ml of 2×YT (comprising 2% glucose, 100 µg/ml ampicillin), cultured at 37°C for 200 r/min until the OD600 reached 0.5, auxiliary phage was added according to the multiplicity of infection of 20:1, and left for 30 min at 37°C, 200 r/min for 30 min. The culture was centrifuged, and the pellet was resuspended with 200 ml of 2×YT (containing 100 µg/ml ampicillin and 50 µg/ml kanamycin), incubated overnight at 37°C, 250 r/min, centrifuged at 8000rpm to obtain the supernatant, added with 5×PEG/NaCl solution, placed on ice for 60 min, centrifuged at 8000rpm for 30 min. The pellet was resuspended in 5 ml of PBS to obtain the single domain heavy chain antibody (VHH) immune library against MSLN, and 10 µL was taken to determine the titer, and the rest were split for storage at -80°C.

### Example 3. Phage Display Panning

### 3.1 Biopanning

A total of 2 rounds of solid-phase panning were performed to screen the phage library, with BCMA his tag protein as positive screening protein and human IgG protein as negative screening protein; BCMA protein concentration of the first round was 4 µg/mL, and the BCMA protein concentration was reduced to 2 µg/mL in the second round of panning . Specific steps are as follows:
At a concentration of 4 µL/mL, 100 µL per well of human BCMA his tag protein was coated on an ELISA plate, placed at 4°C overnight, and at the same time coated with a negative mesh of human IgG protein. The next day, 200 µL of 3% BSA was added to each of the 5 wells, and the cells were blocked at room temperature for 2 hours. Two hours later, the cells were washed three times with PBST (comprising 0.05% Tween 20 in PBS). After the plate was washed, 100 µL phage pre-blocked with 5% skim milk (2-3 × 10¹¹ tfu immunized camelid nanobody phage display gene library) was added, left for 1.5 hours at room temperature, and then the supernatant after negative screening was transferred to the target antigen coated well for 1.5 hours at room temperature. The plate was washed with PBST (comprising 0.05% Tween 20 in PBS) for 12 times to wash out the unbound phage. The phage specifically bound to BCMA his tag was dissociated with Glycine (SIGMA), and the eluted phage was neutralized by Tris (Invitrogen, 1 M, pH 8.0) and infected with TG1 in logarithmic phase. After expansion, the next round of "adsorption-elution" was carried out. Finally, the eluted phages were immersed in TG1, and IPTG (Thermo) was used to induce TG1 to express nanobodies.

### 3.2 Clone Screening

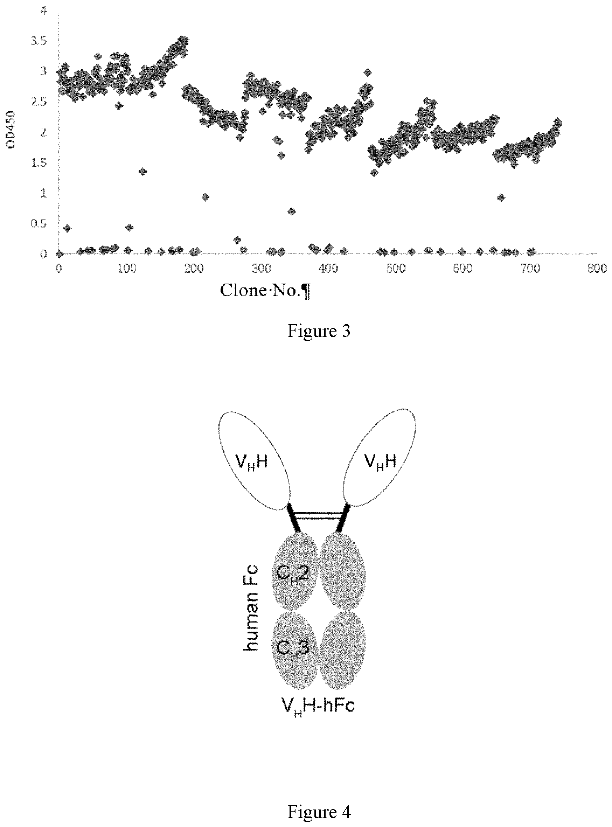

Target antigen-specific clones were identified by ELISA and FACS techniques (see Figure 3). The ELISA plate was coated with BCMA his, and the supernatant was taken for ELISA detection. Figure 3 shows the results of BCMA full-length protein screening by ELISA, and some positive clones were selected for sequencing. After sequence analysis, clones with different CDR3 regions were selected for re-induction, and the binding to CHO-K1/BCMA cell line overexpressing human BCMA protein and RPMI8226 cell line were detected by FACS. Finally, 27 clones specific to the human BCMA target antigen were screened out, as shown in Table 1.

ELISA method: TG1 strains from a single output phage clone were induced to grow overnight in a 96-well deep-well plate. In order to identify clones bound to antigenic proteins, a 96-well ELISA microtiter plate was coated with recombinant human BCMA his tag protein and PD-1 his control protein in coating buffer at 4°C overnight, and then the plate was blocked with blocking buffer. After blocking, approximately 100 µL/well of phage supernatant from overnight cell cultures was added to the plate and incubated for 1 hour at room temperature. Plates were washed four times and HRP-conjugated anti-c-myc antibody was added to the plate and incubated for 30 minutes at room temperature. Plates were washed five more times and substrate solution was added to wells for color development. The absorbance of each well at 450 nm were measured.

FACS method: the cloned supernatant was taken and added to a 96-well plate containing 5×10⁵ CHO-K1/BCMA cells or RPMI8226 cells, incubated at 4°C for 40 minutes; centrifuged at 220g for 5 minutes, the supernatant was discarded, and 200 µL pre-cooled DPBS was added to each well to resuspend, then centrifuged and the supernatant was discarded; 150µL 2.5µg/mL biotin-anti-his antibody (GenScript, A00186-100) was added to each well to resuspend the cells and incubated at 4°C for 40 min; after washing with pre-cooled DPBS one time, 150µL PE Streptavidin (Biolegend) diluted with DPBS according to 1:500 was added to each well, cells were resuspended, and incubated at 4° C for 30 min. After 30 minutes, the cell were centrifuged at 220g for 5 minutes at 4°C, the supernatant was discarded, 200 µL pre-cooled DPBS washing solution was added to each well, the cells were resuspended by gently pipetting with a multichannel pipette, centrifuged at 220g for 5 minutes, the supernatant was discarded, and the washing step was repeated one time. After 2 times of washing, the cells were resuspended in DPBS and detected by a flow cytometry (Beckman, Cytoflex).

### Example 4. Screening of Optimal Clones

### 4.1 Expression and purification of optimal clones

The sequence of the screened nanobody VHH was inserted into the pCDNA3.4-IgG4 plasmid vector, constructed into a format of VHH-hlgG4 (Figure 4), and expressed through the ExpiCHO^{™} (Thermo Fisher) expression system. After expression for one week, the supernatant was collected and purified by protein A (GE), and then the protein quality was detected by Nanodrop, and the protein purity was detected by HPLC. As shown in Table 1, the obtained antibody purity (with a buffer of PBS of pH 7.4) and yield meet the requirement of subsequent experiments.

**Table 1. QC test results of 27 purified nanobodies**

| Antibody No. | lable | **pI** | **MW (kDa)** | **Cell** | Total amount (mg) | **HPLC (%)** |
|---|---|---|---|---|---|---|
| NB-1 | IgG4 | 6.4 | 77.7 | ExpiCHO | 5 | 96.4 |
| NB-27 | IgG4 | 6.3 | 77.5 | ExpiCHO | 3.7 | 93.8 |
| NB-29 | IgG4 | 6 | 77.2 | ExpiCHO | 2.9 | 89.8 |
| NB-34 | IgG4 | 6.4 | 78.2 | ExpiCHO | 4.8 | 96.5 |
| NB-36 | IgG4 | 7.1 | 77.9 | ExpiCHO | 3.6 | 100 |
| NB-51 | IgG4 | 5.9 | 77.4 | ExpiCHO | 4.4 | 96 |
| NB-53 | IgG4 | 6.3 | 77 | ExpiCHO | 3.9 | 92.8 |
| NB-65 | IgG4 | 6.1 | 79.3 | ExpiCHO | 3.5 | 93.8 |
| NB-67 | IgG4 | 6.7 | 78.1 | ExpiCHO | 2.8 | 97.3 |
| NB-71 | IgG4 | 5.9 | 77.5 | ExpiCHO | 0.8 | 59.6 |
| NB-79 | IgG4 | 6.37 | 78.5 | ExpiCHO | 3.9 | 100 |
| NB-82 | IgG4 | 6.4 | 78.1 | ExpiCHO | 2.1 | 95.1 |
| NB-83 | IgG4 | 5.8 | 78 | ExpiCHO | 4.5 | 96.4 |
| NB-84 | IgG4 | 6.6 | 78.2 | ExpiCHO | 1.5 | 98.3 |
| NB-90 | IgG4 | 6 | 78.3 | ExpiCHO | 1.6 | 98.6 |
| NB-100 | IgG4 | 6.6 | 78.2 | ExpiCHO | 1.8 | 98 |
| NB-102 | IgG4 | 6.1 | 78.9 | ExpiCHO | 1.5 | 95.8 |
| NB-122 | IgG4 | 6 | 78.2 | ExpiCHO | 1.8 | 88.4 |
| NB-170 | IgG4 | 5.94 | 77.54 | ExpiCHO-S | 14.3 | 82.2 |
| NB-192 | IgG4 | 6.4 | 78.3 | ExpiCHO | 1.3 | 95.2 |
| NB-216 | IgG4 | 6.3 | 77.9 | ExpiCHO | 1.6 | 100 |
| NB-217 | IgG4 | 5.8 | 78.3 | ExpiCHO | 0.5 | 85.3 |
| NB-222 | IgG4 | 6.4 | 77.9 | ExpiCHO | 13.9 | 98.4 |
| NB-257 | IgG4 | 7.1 | 78.6 | ExpiCHO | 0.9 | 97.2 |
| NB-265 | IgG4 | 6.3 | 77.2 | ExpiCHO | 1.1 | 96.6 |
| NB-352 | IgG4 | 6.29 | 78.4 | ExpiCHO-S | 12.7 | 97.97 |
| NB-367 | IgG4 | 5.9 | 78.4 | ExpiCHO | 1 | 98.6 |

### Example 5. Affinity assay of optimal clone

### 5.1 Ligand Blocking Assay

### 5.1.1 Anti-BCMA antibody competitively blocked the binding of two ligands (APRIL, BAFF) to human BCMA by ELISA

Plates (Corning) were coated with 2 µg/mL human BCMA his tag protein (Acrobiosystems, BCA-H522y) overnight at 4°C. After blocking and washing, 50 µL diluted antibodies (78 µg/mL, 19.5 µg/mL, 4.875 µg/mL, 1.218 µg/mL) and 50 µL biotin-labeled 1 µg/mL APRIL (Acrobiosystems, APL-H82F5) or 1.25µg/mL BAFF (Acrobiosystems, BAF-H82F3) ligand were added to each well, and incubated at 37°C for 1 hour. After washing 3 times, 100 µL HRP-labeled streptavidin (Biolegend, 405210) diluted 1:2000 was added to each well, and incubated at 37° C for 30 minutes. After washing, 100 µL TMB substrate (Abeam, ab171524) was added to each well and developed for 5 minutes and the reaction was stopped by 3M HCl. The absorbance value at 450 nM was read using a microplate reader (Tecan, SPARK). The results showed that all 27 anti-BCMA antibodies could compete for the binding of the two ligands APRIL and BAFF to BCMA. The experimental results of some of the anti-BCMA antibodies are shown in Table 2.

**Table 2. Ligand Competition Results by ELISA (OD450)**

| Biotin-APRIL ligand competition ELISA (OD450) | | | | | Biotin-BAFF ligand competition ELISA (OD450) | | | |
|---|---|---|---|---|---|---|---|---|
| Antibod y | 78 µg/m L | 19.5 µg/mL | 4.875 µg/mL | 1.218 µg/mL | 78 µg/m L | 19.5 µg/mL | 4.875 µg/mL | 1.218 µg/mL |
| NB-1 | 0.17 | 0.41 | 1.23 | 3.10 | 0.04 | 0.03 | 0.26 | 3.04 |
| NB-27 | 0.14 | 0.43 | 1.52 | 3.01 | 0.02 | 0.03 | 0.62 | 3.13 |
| NB-29 | 0.14 | 0.40 | 1.39 | 2.97 | 0.01 | 0.02 | 0.74 | 3.22 |
| NB-34 | 0.15 | 0.47 | 1.52 | 2.89 | 0.02 | 0.06 | 0.86 | 3.28 |
| NB-36 | 0.12 | 0.27 | 1.08 | 2.91 | 0.02 | 0.02 | 0.52 | 3.22 |
| NB-51 | 0.17 | 0.30 | 1.41 | 2.92 | 0.02 | 0.04 | 1.24 | 3.21 |
| NB-53 | 0.42 | 0.92 | 1.62 | 2.86 | 0.02 | 0.07 | 0.90 | 3.22 |
| NB-65 | 0.14 | 0.37 | 1.44 | 2.86 | 0.02 | 0.03 | 0.80 | 3.22 |
| NB-67 | 0.08 | 0.32 | 1.49 | 2.89 | 0.02 | 0.03 | 0.82 | 3.23 |
| NB-71 | 0.03 | 0.19 | 2.53 | 2.93 | 0.02 | 0.03 | 2.69 | 3.26 |
| NB-82 | 0.10 | 0.38 | 1.62 | 2.92 | 0.02 | 0.03 | 0.91 | 3.22 |
| NB-83 | 0.09 | 0.45 | 1.41 | 3.06 | 0.02 | 0.03 | 0.79 | 3.61 |
| NB-84 | 0.22 | 0.69 | 1.60 | 3.21 | 0.03 | 0.03 | 0.23 | 3.33 |
| NB-90 | 0.17 | 0.48 | 1.59 | 2.96 | 0.02 | 0.02 | 0.30 | 3.12 |
| NB-100 | 0.08 | 0.37 | 1.41 | 3.14 | 0.01 | 0.02 | 0.83 | 3.30 |
| NB-102 | 0.10 | 0.41 | 1.60 | 3.10 | 0.01 | 0.07 | 0.85 | 3.36 |
| NB-122 | 0.08 | 0.25 | 1.14 | 3.10 | 0.01 | 0.02 | 0.79 | 3.38 |
| NB-192 | 0.04 | 0.13 | 1.18 | 3.07 | 0.01 | 0.03 | 1.21 | 3.33 |
| NB-216 | 0.07 | 0.35 | 1.73 | 3.07 | 0.01 | 0.05 | 0.94 | 3.34 |
| NB-217 | 0.05 | 0.43 | 2.08 | 3.12 | 0.01 | 0.06 | 1.60 | 3.33 |
| NB-257 | 0.05 | 0.24 | 1.59 | 2.90 | 0.01 | 0.02 | 0.97 | 3.29 |
| NB-265 | 0.10 | 0.27 | 1.12 | 2.78 | 0.02 | 0.02 | 0.76 | 3.25 |
| NB-367 | 0.12 | 0.41 | 1.04 | 2.68 | 0.01 | 0.02 | 0.76 | 3.42 |
| BMK1 | 0.05 | 0.31 | 2.90 | 3.10 | 0.03 | 0.31 | 3.32 | 3.39 |
| Isotype-2 | 3.04 | 2.99 | 3.07 | 2.89 | 3.24 | 3.28 | 3.29 | 3.38 |

See Table 6 for the amino acid sequences of BMK1 and Isotype-2 in Table 2.

### 5.1.2 Competitive blocking activity of anti-BCMA antibodies against APRIL ligands as determined by FACS

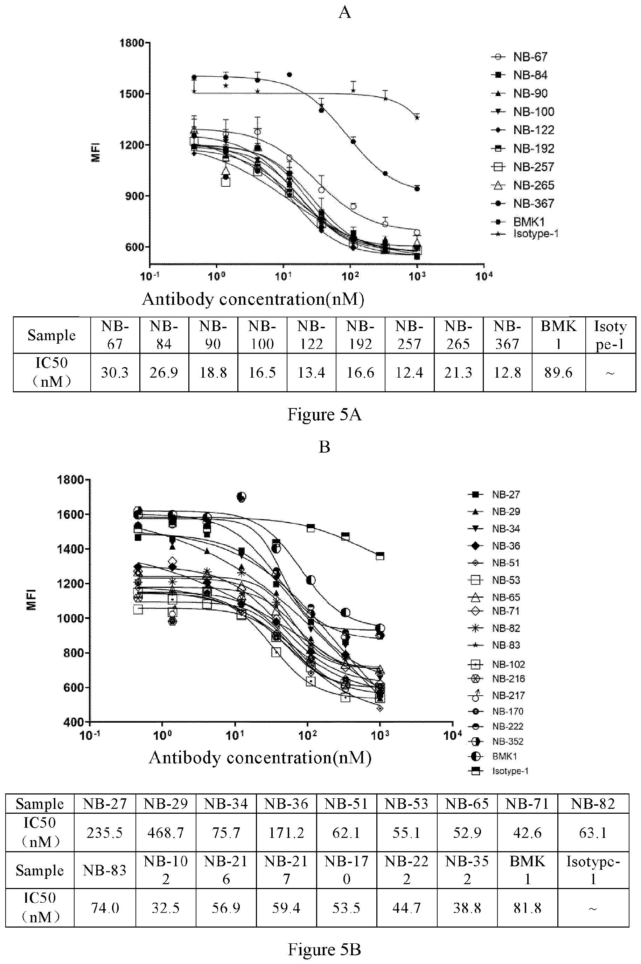

In a 96-well non-adsorption deep-well plate, the purified antibody and the control antibody were diluted to 1000 nM with DPBS, and then serially diluted to 8 concentration with 3-fold dilution . Biotin-APRIL was diluted to 4 µg/mL with DPBS. 50 µL the antibody diluted in the previous step or DPBS (Blank, Blank-PE tube), respectively, was added to the wells of a non-adsorption 96-well plate having 5×10⁵ CHO-K1/BCMA cells that overexpressed human BCMA, and then the 50 µL diluted APRIL in the previous step was added and mixed, incubated at 4°C for 40 min. After washing once with pre-cooled DPBS, 150 µL PE Streptavidin (Biolegend, 405210) diluted with DPBS at 1:500 was added to each well, the cells were resuspended, and incubated at 4° C for 30 min. After 30 minutes, centrifuged at 220g for 5 minutes at 4°C, the supernatant was discarded, 200 µL pre-cooled DPBS washing solution was added to each well, the cells were resuspended by gently pipetting with a multichannel pipette, centrifuged at 220g for 5 minutes, the supernatant was discarded, and the washing step was repeated one time. After 2 time of washing, the cells were resuspended in DPBS and detected by a flow analyzer (Beckman, Cytoflex). The results are shown in Figure 5. Our VHH-hlgG4Fc antibody competed for the binding of the ligand biotin-APRIL to CHO-K1/BCMA cells in a dose-dependent manner, and the higher the antibody concentration, the weaker the ligand-binding signal. Curve fitting was performed by Graphprism software to calculate the IC50 of the antibody, and the results are shown in the table in Figure 5.

### 5.2 Homologous genes (across species) and homologs (across families) screening

### 5.2.1 Cross-reactivity with cynomolgus BCMA and murine BCMA:

Cross-reactivity was determined by ELISA. Plates (Corning) were coated with 2 µg/mLcynomolgus BCMA (Acrobiosystems, BCA-C52H7-100ug) and mouse BCMA (Acrobiosystems, BCA-M52H3) overnight at 4°C. After blocking and washing, 10 µg/mL, 2 µg/mL, and 0.4 µg/mL antibodies were added to BCMA antigen-coated well plates, respectively, and incubated at 37°C for 1 hour. After washing 3 times, 100 µL HRP-labeled goat anti-human IgG Fc antibody (Bethyl, A80-104P) diluted 1:10000 was added to each well, and incubated at 37° C for 30 minutes. After washing, 100 µL TMB substrate (Abeam) was added to each well for 5 minutes and the reaction was stopped with 2M HCl. The absorbance value at 450 nM was read using a microplate reader (Tecan, SPARK).

The results of the cross-species experiments showed that all 27 anti-BCMA antibodies did not bind to cynomolgus BCMA, but most of which bound to murine BCMA (Table 3).

### 5.2.2 Cross-reactivity with human BCMA homologous proteins TACI, BAFFR:

Cross-reactivity was determined by ELISA. Twenty seven test antibodies and BMK control antibody, anti-TACI antibody (Abeam, ab89744), anti-BAFFR antibodies (Abeam, ab16232) were diluted with coating solution to 5µg/mL in the plate (Corning), added to 96-well plates with 100µL/well respectively, and incubated overnight at 4°C. After blocking and washing, 100 µL 4 µg/mL biotin-TACI (Acrobiosystems, TAI-H82F6-25ug), biotin-BAFFR (Acrobiosystems, BAR-H5257-100ug) or control protein were added to the plates coated with different antibodies, respectively, and incubated at 37° C for 1 hour. After washing 3 times, 100 µL 1:2000 diluted HRP Streptavidin (Biolegend, 405210) was added to each well, and then added to the sample wells with 100 µL per well, and incubated at 37° C for 30 minutes. After washing, 100 µL TMB substrate (Abeam, ab171524) was added to each well for 5 minutes and the reaction was stopped with 3M HCl. The absorbance value at 450 nM was read using a microplate reader (Tecan, SPARK). The results showed that all 27 anti-BCMA antibodies had no obvious cross-reaction with the homologous proteins human TACI and BAFFR (Table 3).

| Table 3. Summary table of homologous proteins and species cross-reactivity results | | | | |
|---|---|---|---|---|
| Samble ID | Homologous protein binding | | Species cross-reactivity results (2µg/mL) | |
| | BAFFR | TACI | Mouse BCMA his tag | Cyno BCMA his tag |
| NB-1 | - | - | + | - |
| NB-27 | - | - | + | - |
| NB-29 | - | - | + | - |
| NB-34 | - | - | + | - |
| NB-36 | - | - | ++ | - |
| NB-51 | - | - | ++ | - |
| NB-53 | - | - | ++ | - |
| NB-65 | - | - | ++ | - |
| NB-67 | - | - | - | - |
| NB-71 | - | - | ++ | - |
| NB-79 | - | - | ++ | - |
| NB-82 | - | - | + | - |
| NB-83 | - | - | - | - |
| NB-84 | - | - | ++ | - |
| NB-90 | - | - | ++ | - |
| NB-100 | - | - | ++ | - |
| NB-102 | - | - | ++ | - |
| NB-122 | - | - | + | - |
| NB-170 | - | - | - | - |
| NB-192 | - | - | + | - |
| NB-216 | - | - | - | - |
| NB-217 | - | - | - | - |
| NB-222 | - | - | - | - |
| NB-257 | - | - | ++ | - |
| NB-265 | - | - | + | - |
| NB-352 | - | - | ++ | - |
| NB-367 | - | - | ++ | - |
| Note: "-" means no binding, "+" means binding (OD450≤ 1.0), "++" means strong binding (OD450>1). | | | | |

### 5.3 Protein level affinity detection

The binding affinity of VHH-hIgG4 antibody to human BCMA his tag protein was detected by surface plasmon resonance (SPR). The process is as follows: Biacore T200 (GE) was used in this experiment, the detection temperature was 25°C, and the buffer was 1×HBS-EP+ (10mM HEPES, 150mM NaCl, 3mM EDTA and 0.05% v/v Surfactant P20, GE). One ug/ml VHH-hlgg4Fc was captured on protein A chip (GE) channels 2, 3, and 4 (FC-2, 3, 4), respectively, and channel 1 (FC-1) was used as a blank control. The capture time was 15-20s, and the flow rate was 10ul/min. FC-1, 2, 3, and 4 were injected with BCMA His 0.375, 0.75, 1.5, 3, 6, and 12 nM, respectively, with a binding time of 180s, a flow rate of 30ul/min, and a dissociation time of 300-3000s (the specific time depends on the sample). The chip surface was regenerated by injecting 10mM Glycine-HCl pH1.5(GE) for 30s. Data processing was performed using BIA evaluation Software 2.0 (GE), with double-reference subtraction of sensorgrams, and kinetic constants were calculated by Langmuir 1: 1 model fitting.

The results are shown by Table 4. We obtained multiple strains of VHH-hIgG4 antibodies against human BCMA with different affinities, with KD ranging from 10⁻⁹ to 10⁻¹² and Kd ranging from 10⁻² to 10⁻⁶.

**Table 4. SPR detection results of different antibodies**

| Antibody | Receptor | **ka (1/Ms)** | **kd (1/s)** | **KD (M)** |
|---|---|---|---|---|
| NB-1 | BCMA/TNFR-His | 5.16E+06 | 1.06E-03 | 2.06E-10 |
| NB-29 | BCMA/TNFR-His | 2.15E+06 | 4.13E-04 | 1.92E-10 |
| NB-34 | BCMA/TNFR-His | 2.21E+06 | 1.75E-05 | 7.91E-12 |
| NB-36 | BCMA/TNFR-His | 2.79E+06 | 8.14E-04 | 2.92E-10 |
| NB-51 | BCMA/TNFR-His | 1.12E+07 | 4.96E-04 | 4.44E-11 |
| NB-53 | BCMA/TNFR-His | 2.26E+06 | 1.79E-02 | 7.92E-09 |
| NB-65 | BCMA/TNFR-His | 9.81E+06 | 1.66E-04 | 1.69E-11 |
| NB-67 | BCMA/TNFR-His | 5.98E+06 | 2.96E-04 | 4.95E-11 |
| NB-71 | BCMA/TNFR-His | 9.53E+06 | 2.68E-03 | 2.81E-10 |
| NB-79 | BCMA/TNFR-His | 5.67E+06 | 1.64E-03 | 2.90E-10 |
| NB-82 | BCMA/TNFR-His | 1.75E+06 | 5.55E-05 | 3.18E-11 |
| NB-83 | BCMA/TNFR-His | 6.17E+06 | 2.99E-04 | 4.84E-11 |
| NB-84 | BCMA/TNFR-His | 5.50E+06 | 6.60E-03 | 1.20E-09 |
| NB-90 | BCMA/TNFR-His | 1.16E+07 | 2.75E-03 | 2.36E-10 |
| NB-100 | BCMA/TNFR-His | 7.72E+06 | 3.36E-03 | 4.35E-10 |
| NB-102 | BCMA/TNFR-His | 6.66E+06 | 3.42E-03 | 5.13E-10 |
| NB-122 | BCMA/TNFR-His | 9.69E+06 | 9.68E-06 | 9.99E-13 |
| NB-170 | BCMA/TNFR-His | 2.28E+06 | 2.15E-04 | 9.44E-11 |
| NB-192 | BCMA/TNFR-His | 2.57E+06 | 2.24E-04 | 8.73E-11 |
| NB-216 | BCMA/TNFR-His | 1.03E+06 | 2.70E-04 | 2.62E-10 |
| NB-217 | BCMA/TNFR-His | 3.12E+06 | 2.08E-03 | 6.67E-10 |
| NB-222 | BCMA/TNFR-His | 9.61E+06 | 7.02E-05 | 7.31E-12 |
| NB-257 | BCMA/TNFR-His | 4.77E+06 | 1.02E-02 | 2.14E-09 |
| NB-265 | BCMA/TNFR-His | 8.43E+06 | 3.92E-04 | 4.65E-11 |
| NB-352 | BCMA/TNFR-His | 6.00E+06 | 1.83E-03 | 3.05E-10 |
| NB-367 | BCMA/TNFR-His | 6.74E+06 | 2.06E-03 | 3.05E-10 |

### 5.4 Detection of Cell level affinity:

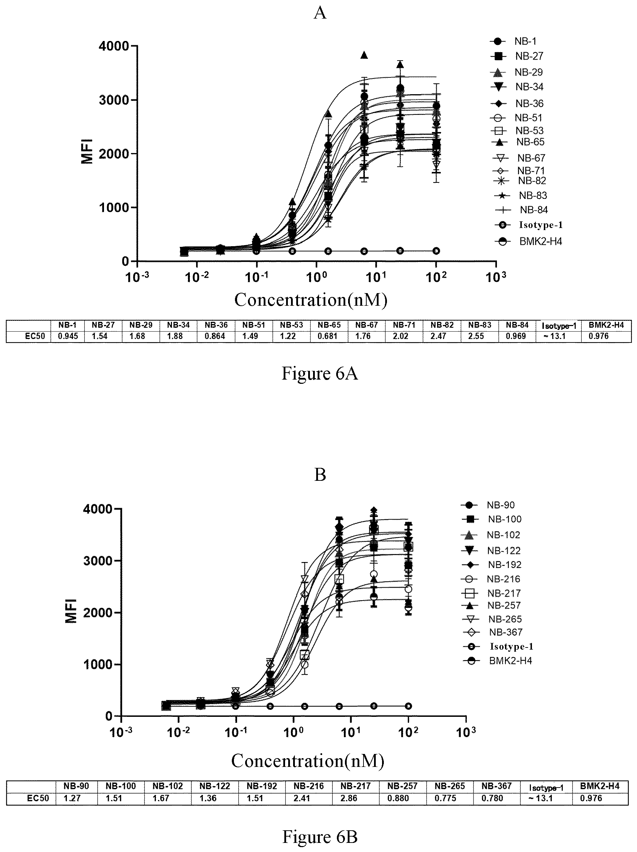

The binding affinity of the anti-BCMA VHH-hIgG4 antibody to target cells (a stable CHO cell line "CHO-K1/BCMA" overexpressing human BCMA) was determined by a cell-based assay. Briefly, the VHH-hlgG4 recombinant antibody was prepared with 100 nM as the initial concentration, and then serially diluted to 8 concentrations with 4-fold dilution. CHO-K1/BCMA cells expressing human BCMA full-length protein were plated in 96-well plates, 5×10⁵ cells per well, and the serially diluted heavy chain antibody (VHH-hlgg4 recombinant antibody) was mixed with CHO-K1/BCMA cells and incubated at 4°C for 40 minutes. After washing, detection secondary antibody anti-human IgG PE (Jackson Immuno Research, Code: 109-117-008) was added and incubated for 30 minutes. After washing, CytoFLEX flow cytometer was used for detection. Curve fitting was performed by Graphprism software and the EC50 of the antibody was calculated. The results are shown in Figure 6, with EC50 ranging from 0.6 to 2.8 nM.

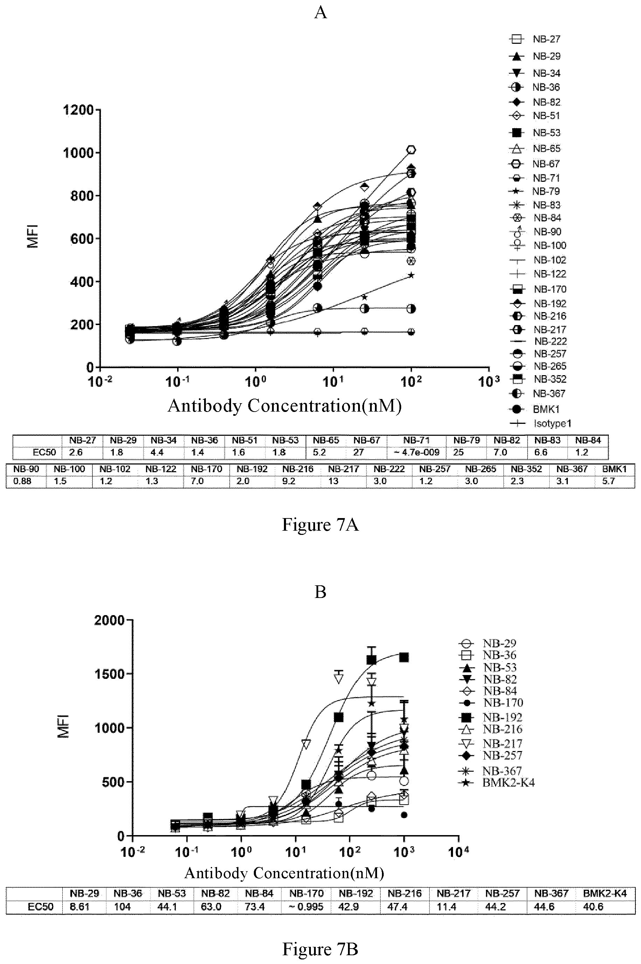

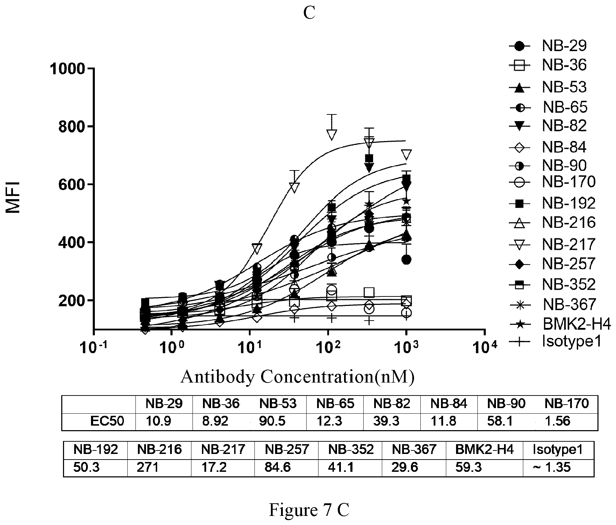

5.5 Binding@@ detection of tumor cells: The serially diluted heavy chain antibody was incubated with U-87MG cell line that does not express BCMA antigen, RPMI8226 cells that overexpress BCMA antigen, Jeko-1 cells, and Daudi cells. The detection antibody is anti-human IgG PE (Jackson Immuno Research, Code: 109-117-008, Lot: 145501). The EC50 of the antibody was calculated by fitting the curve. Results as shown in A, B and C of Figure 7, the binding affinity of each antibody to RPMI8226 cells ranged from 0.8 nM to 7 nM, and the binding affinity of each antibody to Jeko-1 cells ranged from 8 nM to 100 nM. The binding affinity of each antibody to Daudi cells ranged from 1.5 to 300 nM. Each antibody showed no binding to U-87MG, which does not express BCMA.

### Example 6. Specificity Assay of Optimal Clone

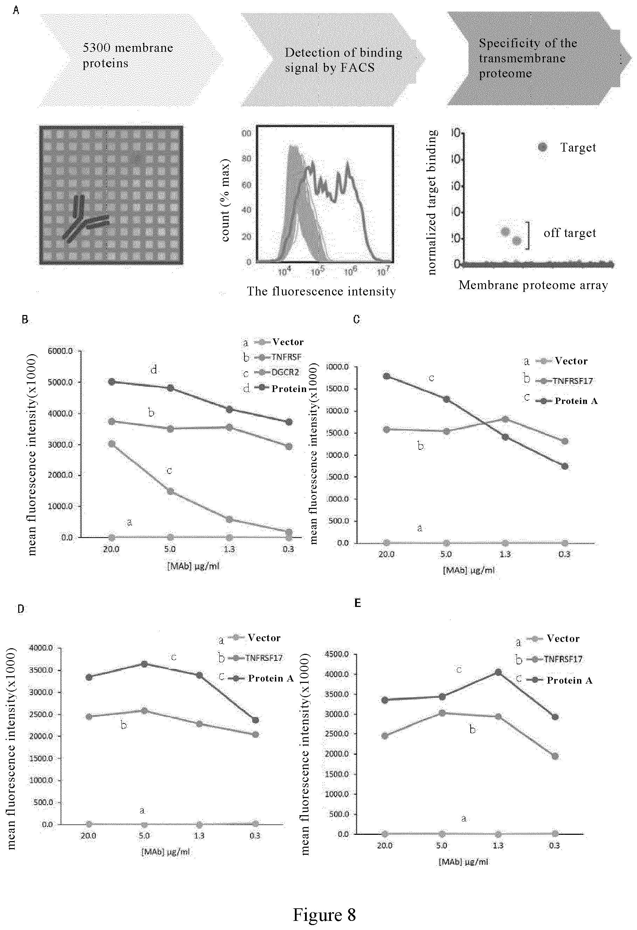

(1) Membrane Proteome Array screening: Membrane Proteome Array is a membrane protein array screening platform developed by Integral molecular company in the United States. They displayed 5,300 different human membrane proteins on the cell surface by transfecting HEK293 cells, and detected antibodies binding signals on these proteins by FACS to evaluated the specificity of the antibody (as shown in Figure 8A). Based on the results of affinity detection and binding of antibodies to various BCMA overexpressing cell lines, 4 representative antibodies were selected for MPA screening. The results showed that the NB-1 antibody not only binds to the target protein BCMA protein, but also has non-specific binding to DGCR2 protein (as shown in B of Figure 8); the other three antibodies including NB-29, NB-257, and NB-367 have good specificity (as shown in C, D, and E of Figure 8, respectively), only specifically binds to the target protein BCMA.
(2)Tissue cross-reaction: This trial was mainly completed by the clinical trial testing center of Shanghai Cell Therapy Group. The purpose of the study was to evaluate whether the optimal antibodies cross-react with 34 kinds of normal human frozen tissues (each tissue derived from 3 different individuals) using immunohistochemical staining for streptavidin conjugated to biotin (Table 5). This test is divided into 3 test groups (NB-29 antibody-Biotin, NB-257 antibody-Biotin, NB-367 antibody-Biotin), recombinant Anti-BCMA antibody EPRBOB-R1-R1-F1-24 (abcam, cat#ab245940), 3 biomarker BMK antibodies (obtained by expression and purification in CHO cells, see Table 6 for sequence and source); and 1 NSG602_OLIGO_31 negative isotype control antibody group (see Table 6 for sequence information).
   a) The test group (NB-29 antibody) showed positive staining in tonsil, thyroid follicular epithelium, liver, duodenum, gastric mucosa, renal tubule and collecting duct.
   b) No specific positive staining was founded in normal human tissues of the test group (NB-257 antibody).
   c) The test group (NB-367 antibody) showed positive staining in thyroid follicular epithelium, liver, duodenum, renal tubule and collecting duct.
   d) In the positive control group, except for the obvious positive staining of tonsil tissue, no specific positive staining was founded with other normal tissues.
   e) BMK1 and BMK3-IgG1 showed no positive binding with 34 kinds of human tissues, and BMK2-H4 showed positive binding with the germinal center of lymphoid region of tonsillar, gastric mucosa and duodenum.
   f) No specific positive staining was founded in normal human tissues of the negative control group.

**Table 5. Summary of tissue cross-reactivity results**

| No. | Organ | Positive control | Negative control | **NB-29** | **NB-25 7** | **NB-36 7** | **BMK1** | **BMK2-H4** | **BMK3-IgG1** |
|---|---|---|---|---|---|---|---|---|---|
| 1 | tonsil | Significa nt positive in Germina 1 center of the lymphati c zone | - | Positive in stroma (3%) | - | - | - | Positive in Germina 1 center of the lymphati c zone | - |
| 2 | liver | - | - | Positive in Liver cells (2-5%) | - | Positiv e in Liver cells (2%) | - | - | - |
| 3 | Thyroid | - | - | Positive in Thyroid follicular epitheliu m (4%) | - | Positiv e in Thyroi d follicul ar epithel ium (20%) | - | Occasio nally positive in myxoid | - |
| 4 | ileum | - | - | - | - | - | - | - | - |
| 5 | Cerebel lum | Positive in molecule layer (5%) | - | 3% | - | - | - | - | - |
| 6 | spleen | - | - | Occasio nally | - | - | - | - | - |
| 7 | heart | - | | - | - | - | - | - | - |
| 8 | artery | - | - | - | - | - | - | - | - |
| 9 | parathy roid | - | - | Glandula r cells (1-2%) | - | Glandu lar cells (3-5%) | - | | |
| 10 | spinal cord | - | - | Occasio nally | - | - | - | - | - |
| 11 | Oviduct | - | - | - | - | - | - | - | - |
| 12 | Duoden um | - | - | Mucosal epitheliu m + duodenal gland (10%) | - | Mucos al epithel ium + duoden al gland (5%) | - | myxoid like positive | - |
| 13 | Pituitar y | - | - | - | - | - | - | - | - |
| 14 | Stomac h | - | - | Gastric mucosa (5%) | - | Gastric mucos a (2%) | - | Occasio nally positive in Mucosa layer | - |
| 15 | Testis | Occasionally positive in seminife rous epitheliu m | - | - | - | - | - | myxoid like positive | - |
| 16 | Colon | - | - | - | - | - | - | Occasio nally positive in Mucosa layer | - |
| 17 | Bladder | - | - | - | - | - | - | - | - |
| 18 | Corpus | - | - | - | - | - | - | Occasio nally diffuse positive | - |
| 19 | Lymph node | - | - | - | NA | NA | - | - | - |
| 20 | Skin | - | - | - | - | - | - | - | - |
| 21 | Placent a | - | - | - | - | - | - | - | - |
| 22 | Eye | - | - | - | - | - | - | - | - |
| 23 | Pancrea s | - | - | Very occasion ally in stroma | - | - | - | - | - |
| 24 | Kidney | - | - | Positive in renal tubules and collectin g ducts (30-40% ) | - | Positiv e in renal tubules and collecti ng ducts (20-25 %) i | - | Occasio nally diffuse positive in stroma | - |
| 25 | Breast | - | - | - | - | - | - | - | - |
| 26 | Adrenal gland | - | - | - | - | - | - | - | - |
| 27 | Vein | - | - | stroma 5% | Very occasio nally positiv e in stroma | stroma 2% | - | - | - |
| 28 | Ureter | - | - | | - | | - | - | - |
| 29 | cervix | - | - | - | - | - | - | - | - |
| 30 | Prostate | Occasio nally positive in stroma | - | - | - | - | Occasio nally positive in stroma | positive in glandular cells | - |
| 31 | Ovary | Occasio nally positive in cortical area | - | - | - | - | Very occasion ally positive in cortical | Occasio nally positive in cortical | - |
| 32 | Skeletal muscle | - | - | - | - | - | - | - | - |
| 33 | Lung | - | - | - | - | - | - | - | - |
| 34 | Brain | - | - | - | - | - | - | - | - |

| | | | | | | | | | |
|---|---|---|---|---|---|---|---|---|---|
| Notes: The various tissues used in this experiment were derived from 3 different individuals. These human tissues have been confirmed to have positive staining for actin, demonstrating tissue viability, exception for blood cells. | | | | | | | | | |

**Table 6. Control antibody and hIgG4 Fc information**

| Name of BMK | Sequence Origin Patent | Applicator | Amino acid sequence |
|---|---|---|---|
| BMK1 (SEQ ID NO: 81) | CN 106795217A | Bluebird bio | DIVLTQSPASLAVSLGKRATINCRASESVSVI GAHLIHWYQQKPGQPPKLLIYLASNLETGVP ARFSGSGSGTDFTLTISSLQAEDAAIYYCLQS RIFPRTFGGGTKLEIKGSTSGSGKPGSGEGST KGQVQLVQSGSELKKPGASVKVSCKASGYT FTDYSINWVRQAPGQGLEWMGWINTETREP AYAYDFRGRFVFSLDTSVSTAYLQISSLKAED TAVYYCARDYSYAMDYWGQGTLVTVSS |
| BMK2-H4 | CN 109153731 A | Legend Biotech | See Seq ID NO: 129 in the patent publication |
| BMK3 (SEQ ID NO: 82) | CN 103562225 A | GSK Plc | QVQLVQSGAEVKKPGSSVKVSCKASGGTFS NYWMHWVRQAPGQGLEWMGATYRGHSDT YYNQKFKGRVTITADKSTSTAYMELSSLRSE DTAVYYCARGAIYDGYDVLDNWGQGTLVT VSSGGGGSGGGGSGGGGSDIQMTQSPSSLSA SVGDRVTITCSASQDISNYLNWYQQKPGKAP KLLIYYTSNLHSGVPSRFSGSGSGTDFTLTISS LQPEDFATYYCQQYRKLPWTFGQGTKLEIK |
| Isotype-1 | CN106146653 A | Nanchang University | See Seq ID NO: 2 in the patent publication |
| Isotype-2 | CN106046152 A. | Nanchang University | See Seq ID NO: 2 in the patent publication |
| NSG602_ OLIGO_3 1 | W2020176815 A2 | ZHEJIANG NANOMAB TECHNOLOG Y CENTER CO. LTD. | See Seq ID NO: 58 in the patent publication |
| Human IgG4 FC (SEQ ID NO: 1) | - | - | EPKSADKTHTCPPCPAPELLGGPSVFLFPPKP KDTLMISRTPEVTCVVVDVSHEDPEVKFNWY VDGVEVHNAKTKPREEQYNSTYRVVSVLTV LHQDWLNGKEYKCKVSNKALPAPIEKTISKA KGQPREPQVYTLPPSREEMTKNQVSLTCLVK GFYPSDIAVEWESNGQPENNYKTTPPVLDSD GSFFLYSKLTVDKSRWQQGNVFSCSVMHEA LHNHYTQKSLSLSPGK |

### Example 7. Summary of antibody variable region sequences

### 7.1 A summary of the CDR region sequences of the antibodies is shown in Table 7 below

**Table 7. Summary of CDR region sequences of anti-BCMA antibodies (IMGT numbering schema**

| Antibo dy Name | CDR1 | SEQ ID NO: | CDR2 | SEQ ID NO: | CDR3 | SEQ ID NO: |
|---|---|---|---|---|---|---|
| NB-1 | GFTFRSSA | 79 | IYPDGRT | 17 | QVGRYVSGVYYQP | 80 |
| NB-27 | GSISSIYA | 7 | ITSGGNT | 9 | NAAPWGSYSPGPGDIAS | 46 |
| NB-29 | GFTFSSSA | 4 | IYSDGST | 23 | AVGAPLVYAAYRP | 33 |
| NB-34 | GSIFSIYA | 6 | VTSEGGT | 25 | NAAPWGSYHPQSDIFS | 44 |
| NB-36 | GFTFSSSA | 4 | IYPDGST | 19 | VIGRRGYAMGDRRL | 51 |
| NB-51 | GFTFSSSA | 4 | IYPDGSA | 18 | QVGRYVSGVDYQP | 50 |
| NB-53 | GFTFSPSA | 3 | IYPDGST | 19 | AIGLAPGYRDPNL | 30 |
| NB-65 | GFTFSSSA | 4 | IYSDGST | 23 | AIGDVRGRWDSRPRHEWT Y | 29 |
| NB-67 | GSIFSIYA | 6 | ITSGSST | 11 | NPAPWGDYTATDFHS | 47 |
| NB-71 | GFTFSSSA | 4 | IYPDGTP | 21 | VVGRRGYAMGDRAL | 52 |
| NB-79 | GFTFSPSA | 3 | IYGDGRA | 14 | GIGRWYDQREKEKDL | 39 |
| NB-82 | GSIFSIYA | 6 | VTTEGST | 26 | NAAPWGSYHPQTDIVS | 45 |
| NB-83 | GSISSIYA | 7 | ITSDNSA | 8 | NVAPWGSYSPGPADIAS | 49 |
| NB-84 | GFTFSSSA | 4 | IYPDGTA | 20 | AIGRWYDQRKKEPGL | 31 |
| NB-90 | GFTFSPSA | 3 | IYGDGNA | 13 | AIGRWYEGRKKEEGL | 32 |
| NB-10 0 | GFTFSSSA | 4 | IYPDGTA | 20 | AVGRWYEQRKKEPGL | 37 |
| NB-10 2 | GFTFSPSA | 3 | IYPDGRT | 17 | GIGRWYENREKEKDL | 41 |
| NB-12 2 | GFTFSSSA | 4 | IYSDGST | 23 | AIGAPDPFDYSGWRRNL | 28 |
| NB-17 0 | EFIFSISA | 2 | ITTDGNS | 12 | DVAPWGYHAPEVGS | 38 |
| NB-19 2 | GFTFSSSA | 4 | IYSDGST | 23 | AIGAPDPFDYSGWRRHL | 27 |
| NB-21 6 | GSIFSIYA | 6 | VTSAGNT | 24 | NSAPWGSYSPGPADVSS | 48 |
| NB-21 7 | GFTFSPSA | 3 | IYSDGST | 23 | AVGIVVPYSEDAWYSTL | 35 |
| NB-22 2 | GSIDRFYA | 5 | ITSGGST | 10 | NAAPWGDYTAHDFAS | 43 |
| NB-25 7 | GFTFSPSA | 3 | IYSDGRA | 22 | GIGRWYEKREKEKGF | 40 |
| NB-26 5 | GFTFSSSA | 4 | IYSDGST | 23 | AVGAPLVYASYRP | 34 |
| NB-35 | GFTFSPSA | 3 | IYGDGRP | 15 | AVGLWYEKREKEKDL | 36 |
| 2 | | | | | | |
| NB-36 7 | GFTFSPSA | 3 | IYGEGST | 16 | GVGRWYEGRQYEHDY | 42 |

### 7.2 A summary of the variable region sequences of the antibodies is shown in Table 8 below

**Table 8. Amino Acid Sequences of 27 Anti-BCMA VHH antibody**

| |
|---|
| NB-1 antibody amino acid sequence (SEQ ID NO: 83) |
| EVQLVESGGGLVQPGGSLRLSCAASGFTFRSSAMSWARQAPGKGLEWVSDIYPDGRTDYAD SIKGRFTMSRDNAKNTVYLLMDSLKPEDTAVYHCQVGRYVSGVYYQPRGQGTQVTVSS |
| NB-27 antibody amino acid sequence (SEQ ID NO: 53) |
| EVQLVESGGGLVQPGGSLRLSCAASGSISSIYAMGWYRQAPGKLRGLVAAITSGGNTFYADS VKGRFTISRDNAKNTVYLQMNSLKPEDTAVYDCNAAPWGSYSPGPGDIASWGQGTQVTVSS |
| NB-29 antibody amino acid sequence (SEQ ID NO: 54) |
| EVQLVESGGGLVQPGESLRLSCAASGFTFSSSAMSWARQAPGKGLEWVSGIYSDGSTYYADS VKGRFTISRDNAKNTVYLQMNSLKPEDTAVYLCAVGAPLVYAAYRPWGQGTQVTVSS |
| NB-34 antibody amino acid sequence (SEQ ID NO: 55) |
| EVQLVESGGGLVQPGGSLRLSCAASGSIFSIYAMGWYRQAPGKQRELVAAVTSEGGTFYRDS VKGRFTISRDNAKNTVYLQMNSLKPEDTAVYQCNAAPWGSYHPQSDIFSWGQGTQVTVSS |
| NB-36 antibody amino acid sequence (SEO ID NO: 561 |
| QVQLVESGGGLVQPGGSLRLSCAASGFTFSSSAMSWARRPPGKGLEWVSDIYPDGSTTYADS MKGRFTISRDNAKNTVYLEIYSLKPEDTAVYYCVIGRRGYAMGDRRLRGQGTQVTVSS |
| NB-51 antibody amino acid sequence (SEQ ID NO: 57) |
| QVQLVESGGGLAQPGGSLRLSCAASGFTFSSSAMSWARQAPGKGLEWVSDIYPDGSAYYAD SMKGRFTISRDNAKNTVYLQMDSLKPEDTAVYHCQVGRYVSGVDYQPRGQGTQVTVSS |
| NB-53 antibody amino acid sequence (SEQ ID NO: 58) |
| QVQLVESGGGLVQPGGSLRLSCAASGFTFSPSAMSWARQAPGKGLEWVSSIYPDGSTLYADS MKGRFTISRDNAKNTVSLQMNSLKPEDTAVYYCAIGLAPGYRDPNLRGQGTQVTVSS |
| NB-65 antibody amino acid sequence (SEQ ID NO: 59) |
| QVQL VESGGGL VQPGGSLRLSCAASGFTFSSSAMSW ARQAPGKELEW VSAIYSDGSTYYAD SVKGRFTISRDNAKNTIYLQMNSLKPEDTANYICAIGDVRGRWDSRPRHEWTYWGQGTQVT VSS |
| NB-67 antibody amino acid sequence (SEQ ID NO: 60) |
| QVQLVESGGGLVQPGGSLRLSCTASGSIFSIYAMGWYRQAPGKPRELVAAITSGSSTFYRDSV KGRFTISRDNAKNTVYLRMNSLKPEDTAVYYCNPAPWGDYTATDFHSWGQGTQVTVSS |
| NB-71 antibody amino acid sequence (SEQ ID NO: 61) |
| EVQLVESGGGLVQPGGSLRLSCAASGFTFSSSAMSWARQAPGKGLEWVSDIYPDGTPTYADS MEGRFTISRDNAKNTVHLQIDSLKPEDTAVYYCVVGRRGYAMGDRALRGQGTQVTVSS |
| NB-79 antibody amino acid sequence (SEQ ID NO: 62) |
| EVQLVESGGGSVQPGGSLRLSCAASGFTFSPSAMSWARQAPGKGFEWVSSIYGDGRAYYAD SMKGRFTISRDNAKHTVYLQMNSLKPEDTALYYCGIGRWYDQREKEKDLWGQGTQVTVSS |
| NB-82 antibody amino acid sequence (SEQ ID NO: 63) |
| EVQLVESGGGLVQPGGSLRLSCAASGSIFSIYAMGWYRQAPGKPRELVAAVTTEGSTFYRDS VKGRFTISRDNAKNAVYLQMNSLKPEDTAVYQCNAAPWGSYHPQTDIVSWGQGTQVTVSS |
| NB-83 antibody amino acid sequence (SEQ ID NO: 64) |
| QVQLVESGGGLVQPGGSLRLSCAASGSISSIYAMGWYRQAPGKQRELVAAITSDNSAFYRDS VKGRFTISRDNAKNTVYLQMDSLKVEDTAVYDCNVAPWGSYSPGPADIASWGQGTQVTVSS |
| NB-84 antibody amino acid sequence (SEQ ID NO: 65) |
| QVQLVESGGGLVQPGGSLRLSCAASGFTFSSSAMSWARQAPGKGLEWVSSIYPDGTAYYAD SMKGRFAISRDNARNTVYLQMNSLKPEDTALYQCAIGRWYDQRKKEPGLWGQGTQVTISS |
| NB-90 antibody amino acid sequence (SEQ ID NO: 66) |
| QVQLVESGGGLVQPGESLRLSCAASGFTFSPSAMSWARQAPGKGLEWVSSIYGDGNAYYSD SMKGRFTISRDNAKNTVYLQMNSLKPEDTALYYCAIGRWYEGRKKEEGLWGQGTQVTVAS |
| NB-100 antibody amino acid sequence (SEQ ID NO: 67) |
| QVQLVESGGGLVQPGGSLRLSCAASGFTFSSSAMSWARQAPGKGLEWVSSIYPDGTAYYAD SMKGRFAISRDNARNTVYLQMNSLKPEDTALYQCAVGRWYEQRKKEPGLWGQGTQVTISS |
| NB-102 antibody amino acid sequence (SEQ ID NO: 68) |
| QVQLVESGGDLVQPGGSLRLSCAASGFTFSPSAMSWARQAPGKGFEWVSTIYPDGRTYYSDS MKGRFTISRDNAKNTVYLQMHSLKPEDTALYYCGIGRWYENREKEKDLWGQGTQVTVSS |
| NB-122 antibody amino acid sequence (SEQ ID NO: 69) |
| QVQLVESGGGLVQPGGSLRLSCAASGFTFSSSAMSWARQAPGMGLERVSSIYSDGSTYYPDS MKGRFTISRDNAKNTVYLQMNSLKPEDTAVYICAIGAPDPFDYSGWRRNLLGQGTQVTVSS |
| NB-170 antibody amino acid sequence (SEQ ID NO: 70) |
| QVQLVESGGGLVQPGGSLRLSCAASEFIFSISAMSWYRQAPGKVLELVSDITTDGNSFYADS MKGRFTISRDNAKNTMYLQMNILKSEDTAAYDCDVAPWGYHAPEVGSWGQGTLVTVSS |
| NB-192 antibody amino acid sequence (SEQ ID NO: 71) |
| QVQLVESGGGLVQPGGSLRLSCAASGFTFSSSAMSWARQAPGKGLERVSSIYSDGSTYYSDS MKGRFTISRDNAKNTVYLQMNSLKPEDTAVYYCAIGAPDPFDYSGWRRHLLGQGTLVTVSS |
| NB-216 antibody amino acid sequence (SEQ ID NO: 72) |
| QVQLVESGGGLVQPGGSLRLTCAASGSIFSIYAMGWYRQAPGKQRELVAAVTSAGNTFYRD SVKGRFTISRDNAKSTVYLQMNSLKPEDTAVYDCNSAPWGSYSPGPADVSSWGQGTLVTVS S |
| NB-217 antibody amino acid sequence (SEQ ID NO: 73) |
| EVQLVESGGGTVQPGGSLRLSCVASGFTFSPSAMSWVRQAPGKGLEWVSGIYSDGSTRYAD SVKGRFTISRDNVKNTVYLQMNSLNPEDTAVYYCAVGIVVPYSEDAWYSTLRGQGTQVTVS S |
| NB-222 antibody amino acid sequence (SEQ ID NO: 74) |
| QVQLVESGGGLVQPGGSLRLSCAASGSIDRFYAMGWYRQAPGKSRTLVTAITSGGSTYYAD SVKGRFTISRDNAKNMLYLQMNSLKPEDTAVYYCNAAPWGDYTAHDFASWGQGTQVTVSS |
| NB-257 antibody amino acid sequence (SEQ ID NO: 75) |
| EVQLVESGGGLVQPGGSLRLSCAASGFTFSPSAMSWARQAPGKGFEWVSSIYSDGRAYYAD TMKGRFTISRDNAKNTVYLQMNSLRPEDTALYYCGIGRWYEKREKEKGFWGQGTQVTVSS |
| NB-265 antibody amino acid sequence (SEQ ID NO: 76) |
| EVQLVESGGGLVQPGESLRLSCAASGFTFSSSAMSWARQAPGKGLEWVSGIYSDGSTYYADS VKGRFTISRDNAKNTVYLQMNSLKPEDTAVYKCAVGAPLVYASYRPWGQGTLVTVSS |
| NB-352 antibody amino acid sequence (SEQ ID NO: 77) |
| QVQLVESGGGLVQPGGSLRLSCAASGFTFSPSAMSWARQAPGKGLEWVASIYGDGRPYYAD SMKGRFTISRDNAKNTVYLQMNSLKPEDTALYYCAVGLWYEKREKEKDLWGQGTQVTVSS |
| NB-367 antibody amino acid sequence (SEQ ID NO: 78) |
| QVQLVESGGGLVQPGGSLRLSCAASGFTFSPSAMSWARRAPGKGLEWVSSIYGEGSTVYAD SMKDRFTISRDNAKNTVYLQMNSLKPEDTAVYYCGVGRWYEGRQYEHDYWGQGTLVTVS S |

## Claims

1. A nanobody, comprising a heavy chain variable region containing CDR1, CDR2 and/or CDR3, and has the function of recognizing and binding to BCMA, and wherein:
the CDR1 is selected from the following group:
(1) as shown by the amino acid sequence of SEQ ID NO: 4, or having substitution, deletion or addition of 1-3 amino acid residues in the amino acid sequence shown by SEQ ID NO: 4;
(2) as shown by the amino acid sequence of SEQ ID NO: 6, or having substitution, deletion or addition of 1-3 amino acid residues in the amino acid sequence shown by SEQ ID NO: 6;
the CDR2 is shown by the amino acid sequence of SEQ ID NO: 23, or has substitution, deletion or addition of 1-3 amino acid residues in the amino acid sequence shown by SEQ ID NO: 23;
the CDR3 is selected from the amino acid sequences shown by SEQ ID NOs: 27-52.

2. The nanobody according to claim 1, wherein when the CDR1 has a substitution of 1-3 amino acid residues in the amino acid sequence shown by SEQ ID NO: 4, the substitution is selected from the group consisting of G1E, T3I, S5R and S6P/I; when the CDR1 has a substitution of 1-3 amino acid residues in the amino acid sequence shown by SEQ ID NO: 6, the substitution is selected from the group consisting of F4S/D, S5R and I6F; and/or, when the CDR2 has a substitution of 1-3 amino acid residues in the amino acid sequence shown by SEQ ID NO: 23, the substitution is selected from the group consisting of I1V, Y2T, S3P/G/T, D4G/E/A, G5S/N, S6R/N/G/T and T7A/P/S;
preferably, the CDR1 is selected from the amino acid sequences shown by SEQ ID NOs: 2-7; the CDR2 is selected from the amino acid sequences shown by SEQ ID NOs: 8-26; and/or the CDR3 is selected from the amino acid sequences shown by SEQ ID NOs:27-52.

3. The nanobody according to claim 1 or 2, wherein the heavy chain variable region comprises CDR1, CDR2 and CDR3 shown by any group of the following groups 1 to 26:
| Group | CDR1 (SEQ ID NO) | CDR2 (SEQ ID NO) | CDR3 (SEQ ID NO) |
|---|---|---|---|
| 1 | 7 | 9 | 46 |
| 2 | 4 | 23 | 33 |
| 3 | 6 | 25 | 44 |
| 4 | 4 | 19 | 51 |
| 5 | 4 | 18 | 50 |
| 6 | 3 | 19 | 30 |
| 7 | 4 | 23 | 29 |
| 8 | 6 | 11 | 47 |
| 9 | 4 | 21 | 52 |
| 10 | 3 | 14 | 39 |
| 11 | 6 | 26 | 45 |
| 12 | 7 | 8 | 49 |
| 13 | 4 | 20 | 31 |
| 14 | 3 | 13 | 32 |
| 15 | 4 | 20 | 37 |
| 16 | 3 | 17 | 41 |
| 17 | 4 | 23 | 28 |
| 18 | 2 | 12 | 38 |
| 19 | 4 | 23 | 27 |
| 20 | 6 | 24 | 48 |
| 21 | 3 | 23 | 35 |
| 22 | 5 | 10 | 43 |
| 23 | 3 | 22 | 40 |
| 24 | 4 | 23 | 34 |
| 25 | 3 | 15 | 36 |
| 26 | 3 | 16 | 42 |

4. The nanobody according to any one of claims 1 to 3, wherein the amino acid sequence of the heavy chain variable region is shown by SEQ ID NOs: 53-78, or have at least 80%, 90%, 95%, 96%, 97%, 98% or 99% identity to the amino acid sequences shown by SEQ ID NOs: 53-78.

5. A BCMA binding molecule, wherein the BCMA binding molecule comprises the nanobody according to any one of claims 1 to 4; preferably, the BCMA binding molecule is a monovalent or multivalent nanobody, bispecific antibody, multispecific antibody, heavy chain antibody or antigen-binding fragment thereof comprising one, two or more of the nanobody according to any one of claims 1 to 4.

6. The BCMA binding molecule according to claim 5, wherein the BCMA binding molecule is a heavy chain antibody; preferably, the amino acid sequence of the Fc of the heavy chain antibody is shown by SEQ ID NO: 1, or has at least 80%, 90%, 95%, 96%, 97%, 98% or 99% identity to the amino acid sequences shown by SEQ ID NO: 1.

7. An isolated nucleic acid, wherein the isolated nucleic acid encodes the nanobody according to any one of claims 1 to 4, or the BCMA binding molecule according to claim 5 or 6.

8. A recombinant expression vector, wherein the recombinant expression vector comprises the isolated nucleic acid according to claim 7; preferably, the backbone of the recombinant expression vector is pCDNA3.4.

9. A transformant, wherein the transformant comprises the isolated nucleic acid according to claim 7, or the recombinant expression vector according to claim 8; preferably, the host of the transformant is a prokaryotic cell or eukaryotic cells; more preferably, the eukaryotic cells are CHO cells.

10. A composition, comprising one, two or more nanobodies according to any one of claims 1 to 4, BCMA binding molecules according to claim 5 or 6, the isolated nucleic acid according to claim 7, the recombinant expression vector according to claim 8 or the transformant according to claim 9; preferably, the composition further includes pharmaceutically acceptable excipients.

11. A BCMA detection agent, wherein the BCMA detection agent comprises the nanobody according to claims 1 to 4, and/or the BCMA binding molecule according to claim 5 or 6; preferably, the BCMA detection reagents are used for flow cytometry.

12. The use of the nanobody according to any one of claims 1 to 4, the BCMA binding molecule according to claim 5 or 6, the isolated nucleic acid according to claim 7, the recombinant expression vector according to claim 8, or the transformant according to claim 9 in the preparation of a medicament for the treatment of a BCMA-related disease; preferably, the disease is a hematological malignancy, more preferably multiple myeloma.
